# EUROPEAN PATENT APPLICATION

(11) **EP 4 549 424 A1**
(43) Date of publication of application: **07.05.2025**
(21) Application number: 23849157.5
(22) Date of filing: 04.07.2023
(51) Int. Cl.: C07C 49/83, C07C 49/825, C07C 49/82, C22B 26/12, C22B 3/40

(54) **NEW LITHIUM EXTRACTING AGENT AND LIQUID ORGANIC MIXTURE THEREOF, AND METHOD FOR EXTRACTING LITHIUM BY MEANS OF EXTRACTION**

(30) Priority: 01.08.2022 CN 202210918111
(71) Applicant: Beijing Salt Lake Technology Development Company Limited, Beijing 100098 (CN)
(72) Inventor: ZENG, Debin, Beijing 100098 (CN)
(74) Representative: Cabinet Becker et Associés
(86) International application number: PCT/CN2023/105665
(87) International publication number: WO 2024/027447

(57) **Abstract**

Provided are a compound of formula (I) which is used as a lithium extracting agent. Further provided are a liquid organic mixture which contains the foregoing compound and is used for extracting lithium by means of extraction, and a method for extracting lithium by means of extraction using the organic mixture.

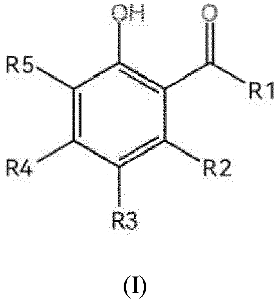

## Description

The present application claims the priority of the Chinese Application No. 202210918111.5 filed on August 1, 2022, the disclosure of which is incorporated herein by reference in its entirety as a part of the present application.

### TECHNICAL FIELD

The present invention relates to the field of lithium extraction technology, specifically to compounds for extracting lithium from an aqueous lithium-containing solution, combinations thereof, and methods of use thereof. More specifically, the present invention relates to extraction compositions having phenolic compounds as lithium extracting agent, and methods for extracting lithium using the extraction compositions.

### BACKGROUND

Lithium is widely used in the fields of batteries, glass, alloys, ceramics, and lubricants, etc. With the explosive growth of the demand for lithium salts in the power battery industry, lithium has been acclaimed as the "energy metal in the 21^{st} century". Lithium is mainly found in solid ores such as spodumene, lepidolite, and lithium clay, as well as in liquid ores such as salt lake brine, oilfield brine, and geothermal water, whereas the development of any type of lithium ores inevitably involves the stage of extracting lithium from an aqueous solution containing alkali and alkaline earth metals. Developing compounds with special selectivity for lithium and their combinations is a key area for efficient lithium extraction.

At present, main methods for extracting lithium salts from an aqueous lithium-containing solution at home and abroad include precipitation, adsorption, ion exchange, electrodialysis membrane, nanofiltration membrane, and solvent extraction methods. The solvent extraction method has advantages such as high efficiency, high selectivity, simple process and equipment, continuous operation, and ease in automatic control. Moreover, existing extraction technologies have been able to achieve the separation of magnesium and lithium, calcium and lithium, and sodium (potassium) and lithium, and can extract lithium from lithium-containing old brine and lithium precipitation mother liquor. Common reagents for lithium extraction include: tributyl phosphate-iron(III) chloride composition, β-diketone-neutral phosphine composition, pyrazolone-TBP composition, crown chemicals, and neutral amides.

The tributyl phosphate-iron (III) chloride composition can extract lithium from a lithium-containing solution with a relatively high chloride ion concentration; and the β-diketone-neutral phosphorus composition can extract lithium from an alkaline lithium-containing solution. The tributyl phosphate-iron (III) chloride composition is highly corrosive to equipments during the extraction; and the crown chemicals lack an industrial application prospect due to their difficult synthesis and high cost. The β-diketone compounds for extracting lithium from an alkaline lithium-containing solution are relatively expensive, and often have significant solution loss due to shorter alkyl chains; and the fluorinated β-diketone compounds that can directly extract lithium from a weakly alkaline aqueous lithium-containing solution have even higher manufacturing cost, which also limits their industrial application to a certain extent. In addition, it has been found that salicylates (such as phenyl salicylate [Chinese Patent Application No. 202210079673.5], and isopropyl salicylate [Tsivadze AY, Bezdomnikov AA, Baulin VE, Demina LI, Birin KP, Baulin DV, Rogacheva YI. A New Extraction System Based on Isopropyl Salicylate and Trioctylphosphine Oxide for Separating Alkali Metals. Molecules. 2022 May 10;27(10):3051. doi: 10.3390/molecules27103051. PMID: 35630527; PMCID: PMC9146891.]) can be used to replace the β-diketones for lithium extraction under alkaline conditions, which are cheaper than β-diketones. However, these compounds will undergo significant hydrolysis and oxidation when used, especially when contacting an alkaline solution, due to the presence of ester groups. On the one hand, it increases the consumption of extracting agents; and on the other hand, it increases the solution loss in aqueous solutions, leading to a high water-treatment cost for prevent and controlling the pollution. More importantly, the disclosed compounds can only extract lithium under highly alkaline conditions (pH > 12) with high alkali consumption and high cost, and thus are difficult to use in an industrial application.

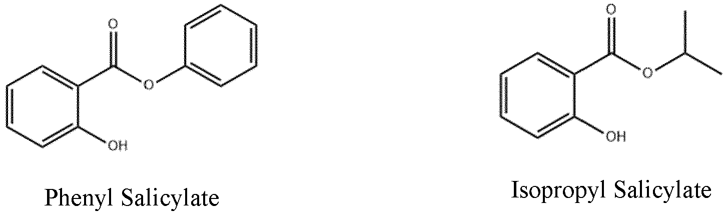

Therefore, there is a need to develop a novel lithium extracting agent, extraction composition, and extraction process to meet the economic and efficiency requirements in industrial application.

### SUMMARY OF THE INVENTION

The inventors have developed a novel lithium extracting agent through intensive study research, overcoming one or more of the aforementioned shortcomings of existing lithium extracting agents.

In an aspect, the present invention provides a lithium extracting agent, which is a compound of Formula (I), 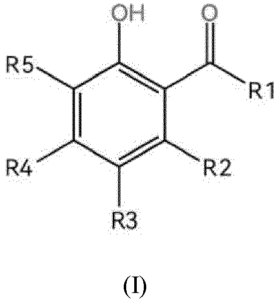
wherein R1 is hydrogen, C₁₋₈ alkyl, C₃₋₆ cycloalkyl, or phenyl, where the C₁₋₈ alkyl, C₃₋₆ cycloalkyl, or phenyl is optionally substituted with one or more substituents selected from the group consisting of hydroxyl, halogen, C₁₋₆ alkyl, C₁₋₆ alkoxy, and nitro, and the C₁₋₆ alkyl or C₁₋₆ alkoxy is optionally further substituted with one or more substituents selected from the group consisting of hydroxyl, halogen, C₁₋₆ alkyl, C₁₋₆ alkoxy, and nitro; and
R2, R3, R4 and R5 are each independently hydrogen, halogen, nitro, benzyloxy, C₁₋₆ alkyl, or C₁₋₁₆ alkoxy, where the benzyloxy, C₁₋₆ alkyl, or C₁₋₁₆ alkoxy is optionally substituted with one or more substituents selected from the group consisting of hydroxyl, halogen, C₁₋₆ alkyl, C₁₋₆ alkoxy, and nitro.

In another aspect, the present invention provides use of the compound of Formula (I) in lithium extraction as lithium extracting agent.

In still another aspect, the present invention provides a liquid organic mixture for extracting lithium by means of extraction, comprising: one or more of the compound of Formula (I), a synergist, and optionally a diluent.

In yet still another aspect, the present invention provides a method for extracting lithium by means of extraction, comprising:
contacting the liquid organic mixture for extracting lithium by means of extraction with an aqueous solution containing lithium ions, allowing the lithium ions in the aqueous solution to enter an organic layer by liquid-liquid layer separation, and collecting the organic layer.

### DETAILED DESCRIPTION OF THE INVENTION

To make the objectives, technical solutions, and advantages of the embodiments of the present invention clearer, the embodiments of the present invention are described clearly and completely below. The embodiments described below are exemplary and are intended to explain the present invention, and should not be understood as limiting the present invention. All other embodiments obtained by a person of ordinary skill in the art without creative effort based on the described embodiments of the present invention should fall within the scope of protection of the present invention.

The present invention can be implemented in other specific forms without departing from the essential attributes of the present invention. It should be understood that, unless otherwise stated, any and all embodiments of the present invention can be combined with any other embodiment, or multiple other embodiments to obtain additional embodiments, without departing from the scope of the present invention. The present invention includes such additional embodiments obtained by combination.

All publications and patents mentioned in the present disclosure are hereby incorporated by reference in their entirety. In the event of any conflict between the uses, or terms used in any publication, or patent incorporated by reference and those used in the present disclosure, the uses and terms of the present disclosure shall prevail.

The section headings used in this document are for organizational purposes only and should not be interpreted as limiting the subject matter described.

Unless otherwise specified, all technical and scientific terms used in this document have their usual meanings in the field to which the subj ect matter claimed pertains. If there are multiple definitions for a term, the definition provided in this document shall prevail.

Unless otherwise indicated in the working examples, or otherwise stated, all numerical values stated in the specification and claims, such as those relating to quantities, should be understood as being modified by the term "about" in all instances. It should also be understood that any numerical range recited in this application is intended to include all subranges and any combinations of the endpoints of that range, or subranges.

The terms "comprise", "contain", or "include" as used herein, mean that the elements preceding the term encompass the elements listed after the term and their equivalents, without excluding unlisted elements. The terms "comprise", "contain", or "include" used in this document can be open-ended, semi-closed, and closed. In other words, said terms also include "consist essentially of", or "consist of."

The term "optional" as used herein means that the described situation may or may not occur. For example, a composition optionally comprising an diluent means that the composition may include a diluent, or may not include a diluent.

The term "extracting agent" as used herein refers to a substance that can not only react with a metal to be extracted (such as, lithium, sodium, or potassium) through coordination chemistry to form a complex extracted into an organic phase, but also undergo a certain chemical reaction so that the extracted metal is stripped from the organic phase into an aqueous phase, thereby achieving the purification or enrichment of metal. Herein, the term "extracting agent" can be used interchangeably with "extractor". For example, a lithium extracting agent or lithium extractor is used to purify or enrich the lithium by means of extraction.

The term "alkyl" as used herein refers to a branched or linear monovalent hydrocarbon group having *n* carbon atoms and *2n+1* hydrogen atoms. Typical alkyl groups include, but are not limited to, methyl, ethyl, propyl, isopropyl, butyl, isobutyl, sec-butyl, tert-butyl, pentyl, isopentyl, neopentyl, hexyl, heptyl, octyl, 2-ethylhexyl, nonyl, decyl, dodecyl, and hexadecyl, etc.

The term "alkoxy" as used herein refers to the alkyl group as defined above, which is attached to a parent structure via an oxygen atom. Typical alkoxy groups include, but are not limited to, methoxy, ethoxy, propoxy, isopropoxy, butoxy, isobutoxy, sec-butoxy, tert-butoxy, pentyloxy, isopentyloxy, neopentyloxy, hexyloxy, heptyloxy, octyloxy, 2-ethylhexyloxy, nonyloxy, decyloxy, dodecyloxy, hexadecyloxy, etc.

The term "cycloalkyl" as used herein refers to a monocyclic or polycyclic group composed only of carbon and hydrogen atoms, which may be saturated or partially unsaturated. In some embodiments, the cycloalkyl is C₃-C₆ cycloalkyl. Illustrative examples of the cycloalkyl groups include, but are not limited to, cyclopropyl, cyclobutyl, cyclopentyl, cyclopentenyl, cyclohexyl, cyclohexenyl, etc. In some preferred embodiments, the cycloalkyl is cyclopentyl or cyclohexyl.

The term "nitro" as used herein refers to a -NO₂ group.

The term "benzyloxy" as used herein refers to a monovalent group PhCH₂O- derived by removing a hydrogen atom from the hydroxyl group of benzyl alcohol.

The term "halo" or "halogen" as used herein refers to fluorine, chlorine, bromine, or iodine. In some embodiments, the "halo" or "halogen" is chlorine or bromine. In some other embodiments, the "halo" or "halogen" is fluorine.

The synergist as used herein refers to an agent which can increase the distribution ratio upon addition into an extracting agent. The term "synergist" as used herein can be used interchangeably with "co-extracting agent."

The diluent as used herein refers to an agent which can improve the physical properties of the extraction organic phase, such as, serving as an organic solvent for the extracting agent, increasing the solubility of the extracting agent in the organic phase, reducing the viscosity of the extracting agent to increase its fluidity, and altering the density of the organic phase.

The present invention is designed to disclose a class of compounds which are stable in properties, inexpensive, and can selectively extract lithium from a lithium-containing aqueous solution, as well as a lithium extraction mixture composed of the same. In particular, it discloses a compound for extracting lithium from liquid lithium resources such as lithium-containing brines with low magnesium and calcium, naturally occurring alkaline lithium-containing brines, and lithium carbonate/lithium phosphate lithium precipitation mother liquors, to replace known β-diketone- and salicylate-based lithium extracting agents, so that the deficiencies in existing lithium extraction technologies such as difficulty in synthesis of extracting agents, high cost, and high solution loss can be overcome.

The present invention is based on the understanding and discovery of the following fundamental principles:
The basic principles of the β-diketone lithium extracting agent interacting with lithium is as follows. The β-diketones exhibit a certain acidity via a keto-enol tautomerism, and form anions in the presence of alkalinity such as hydroxide (OH⁻), carbonate (CO₃²⁻), and bicarbonate (HCO₃⁻) to combine with lithium ions (Li⁺) to form neutral complexes. However, when the β-diketone extracting agents alone are used to extract lithium from an aqueous solution, both the extraction effect and the separation capability from other alkali metals are very limited. At present, the main approach is to enhance the effect by adding a neutral phosphorus synergist, and the acting mechanism thereof is as follows.

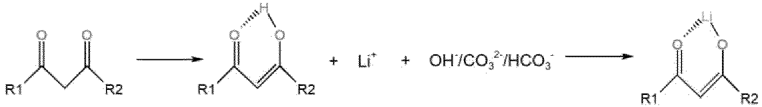

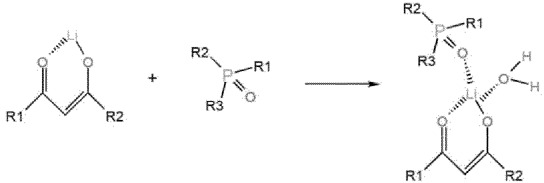

As seen, there are three keys for using β-diketones as lithium extracting agent: 1) they are highly capable to form keto-enol structures; 2) the formed keto-enol structures have high acidity so that they can react with the alkalinity in the aqueous solution containing lithium ions to form anions; and 3) neutral phosphorus hydrogen-bonding acceptors (HBA) enhance the acidity of active hydrogens in the keto-enol structures and the coordination of lithium ions.

Recently, it has been reported that salicylates (such as phenyl salicylate and isopropyl salicylate) [Chinese Patent Application No: CN114438343A; and Foreign Literature: Aslan Yu. Tsivadze et al., Molecules 2022, 27(10), 3051] are also capable of extracting lithium under strongly alkaline conditions. However, the ester groups are prone to hydrolysis and oxidation under alkaline conditions, and the alkali adjustment of the pre-extraction solution and the water treatment of the post-extraction solution greatly increase the production costs, which restrict their industrial application prospects.

The inventors have found through extensive research that the structure of β-diketones and the ester group structures in salicylates are not necessary for ensuring the lithium extraction performance. However, the hydrogen bonding ring structure formed by hydroxyl and ketone groups is the key to coordinate with lithium ions. **In** the structure of alkanes, the hydrogen bonding ring structure formed by hydroxyl and ketone groups is the keto-enol structure of β-diketones. And in the aromatic structures containing phenyl ring, a phenolic hydroxyl group can be used to replace the keto-enol structure to present an acidity. When a ketone group exists at the ortho position of the phenolic hydroxyl group, a hydrogen bonding ring structure formed by hydroxyl and ketone groups as shown in the following formula can also be constituted. 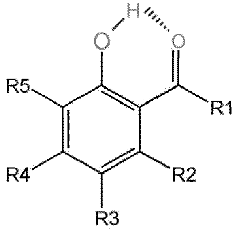 Compared to the β-diketones, a compound having the hydrogen bonding ring structure as shown in the above formula has 100% of acidic structure, which is much higher than the proportion of keto-enol structures in β-diketones that play a role in lithium coordination. Moreover, the modification of the structures with acidity from the phenolic hydroxyl groups is much easier than that of β-diketones, and can be made by changing R2, R3, R4, and R5. When R1 is alkyl or phenyl, it exhibits very good stability under alkaline conditions. Furthermore, the modifications of R2, R3, R4, and R5 are much richer than the modification of R1 alone to improve the performance of such lithium extracting agents. In particular, when one of R2, R3, R4, and R5 is alkoxy, the chain length can be adjusted by the etherification reaction of the phenolic hydroxyl, thereby obtaining an inexpensive lithium extracting agent.

The present invention discloses compounds with structure as shown in the following formula, which are simple to prepare, inexpensive, and have an ability of separating lithium ions from an aqueous solution: 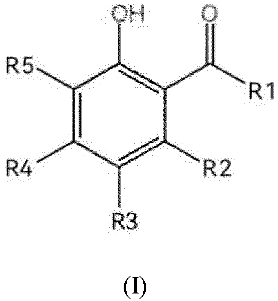
wherein R1 is H, C₁₋₈ alkyl, C₃₋₆ cycloalkyl, or phenyl, where the C₁₋₈ alkyl, C₃₋₆ cycloalkyl, or phenyl is optionally substituted with one or more substituents selected from the group consisting of hydroxyl, halogen, C₁₋₆ alkyl, phenyl, C₁₋₆ alkoxy, or nitro, and the C₁₋₆ alkyl or C₁₋₆ alkoxy is optionally further substituted with one or more substituents selected from the group consisting of hydroxyl, halogen, C₁₋₆ alkyl, C₁₋₆ alkoxy, or nitro;
R2, R3, R4 and R5 are each independently hydrogen, halogen, nitro, benzyloxy, C₁₋₆ alkyl, or C₁₋₁₆ alkoxy, where the benzyloxy, C₁₋₆ alkyl, or C₁₋₁₆ alkoxy is optionally substituted with one or more substituents selected from the group consisting of hydroxyl, halogen, C₁₋₆ alkyl, C₁₋₆ alkoxy, and nitro.

For example, the compound of Formula (I) comprises, without limitation: 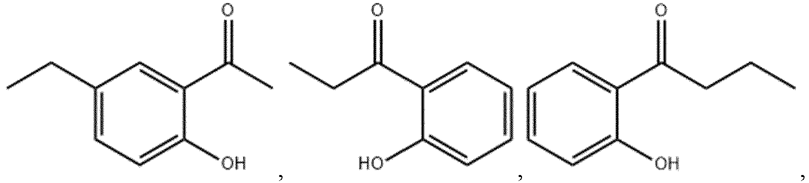 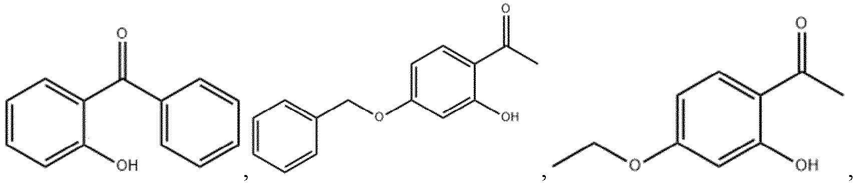 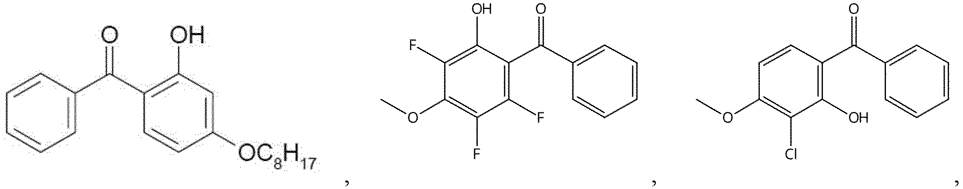 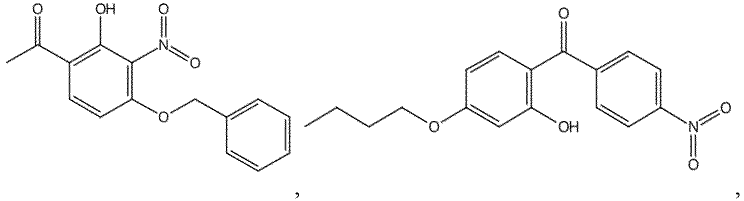 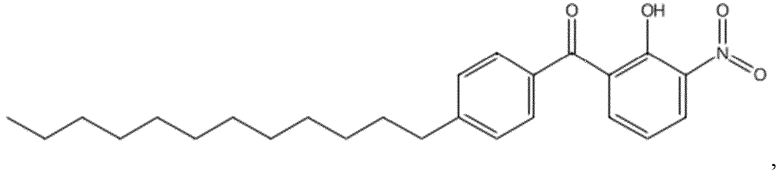 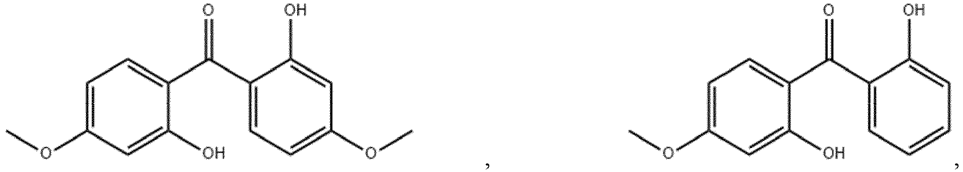 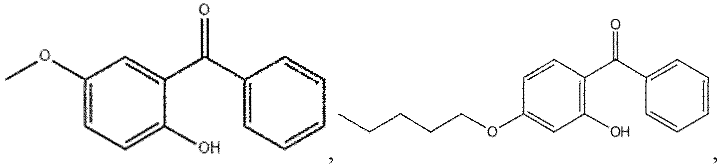 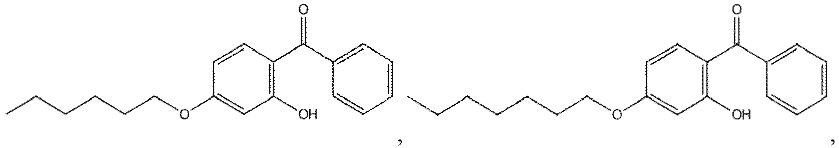 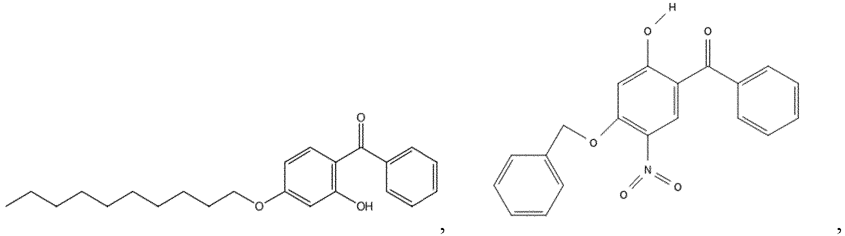 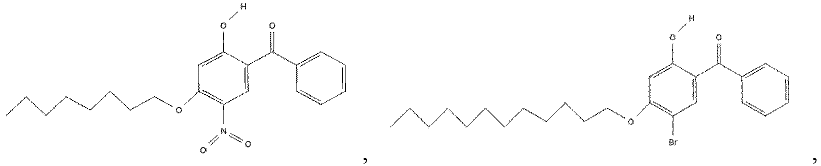 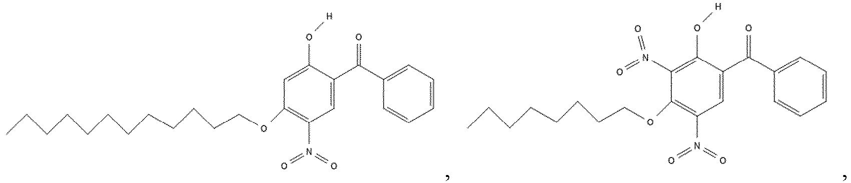 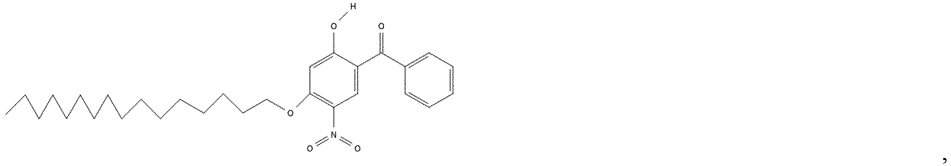 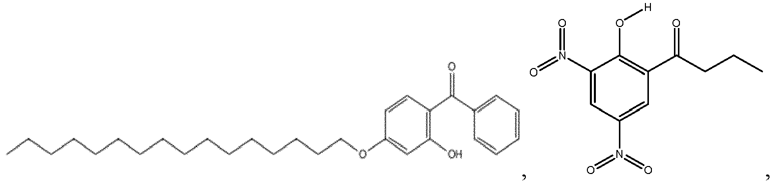 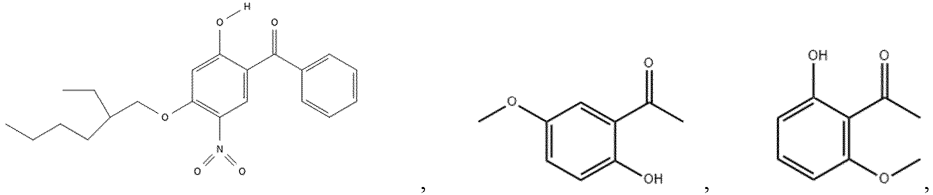 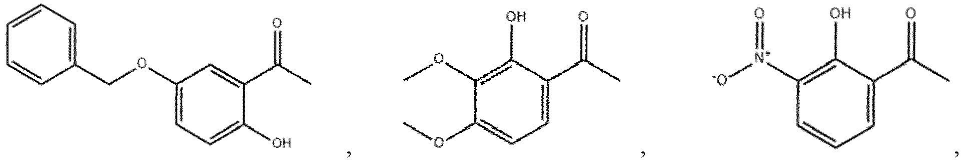 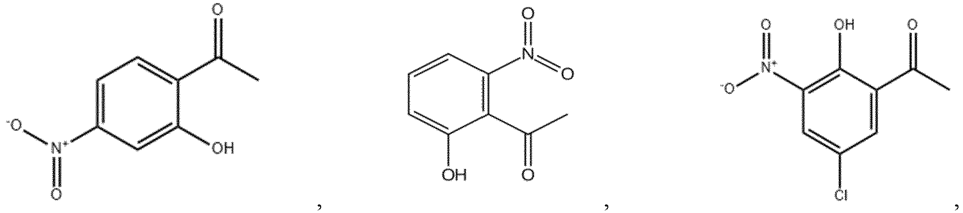 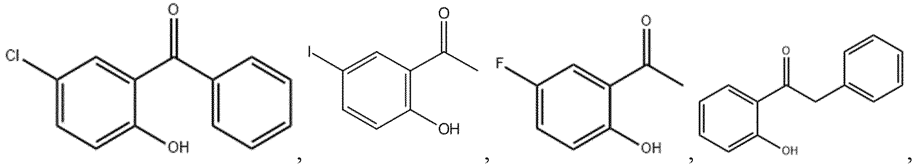 and

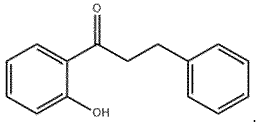

In overall consideration of the economy, oil solubility, loss in water solubility, and material availability in the preparation of lithium extracting agent, as well as the atomic economy of synthesis, the lithium extracting agent of Formula (I) can be optimized by selecting R1-R5.

In an embodiment, R1 is C₁₋₆ alkyl or phenyl, where the C₁₋₆ alkyl or phenyl is optionally substituted with one or more substituents selected from the group consisting of hydroxy, halogen, C₁₋₆ alkyl, phenyl, or C₁₋₆ alkoxy. For example, R1 is methyl, ethyl, benzyl, phenethyl, propyl, phenyl, or 2-hydroxy-4-methoxyphenyl. In a preferred embodiment, R1 is methyl, ethyl, propyl, benzyl, or phenyl.

In an embodiment, at least one of R2, R3, R4, and R5 is nitro or halogen. In a preferred embodiment, at least one of R2, R3, R4, and R5 is nitro. It is believed that the introduction of electron-withdrawing groups such as nitro or halogen on the phenyl ring can increase the acidity of the phenolic hydroxyl group.

In an embodiment, one of R2, R3, R4, and R5 in the compound of Formula (I) is benzyloxy group, or an unsubstituted or halogen-substituted C₁₋₁₆ alkoxy group. It is believed that the introduction of benzyloxy or alkoxy on the phenyl ring can increase the oil solubility and decrease the water solubility of the compound of Formula (I).

In a preferred embodiment, one of R2, R3, R4, and R5 in the compound of Formula (I) is an unsubstituted or halogen-substituted C₆₋₁₆ alkoxy chain, more preferably, R4 in the compound of Formula (I) is an unsubstituted or halogen-substituted C₆₋₁₆ alkoxy chain. At that time, the compound of Formula (I) has suitable oil solubility and low water solubility, with minimal solution loss in the aqueous solution during the lithium extraction process, and the materials are widely available and inexpensive. For example, it can be synthesized from 2,4-dihydroxyacetophenone or 2,4-dihydroxydibenzophenone and C₆₋₁₄ haloalkane by Williamson ether synthesis reaction. The synthesis process has mild conditions, low material costs, and high yield. Preferably, the haloalkane used is hexyl bromide, heptyl bromide, octyl bromide, isooctyl bromide, nonyl bromide, decyl bromide, undecyl bromide, dodecyl bromide, tridecyl bromide, tetradecyl bromide, hexadecyl bromide, benzyl chloride, and/or benzyl bromide.

Furthermore, a substituent modification can be made on the phenyl ring based on the preferred compound of Formula (I) containing a C₆₋₁₆ alkoxy chain to achieve the purpose of adjusting the acidity of the phenolic hydroxyl group, so that it can extract lithium from an aqueous solution over a wide pH range. Preferably, the modifying group on the phenyl ring includes electron-withdrawing groups such as fluorine, chlorine, bromine, iodine, and nitro. Specifically, the preferred compound of Formula (I) containing a C₆₋₁₆ alkoxy chain can react with bromine at low temperature to obtain mono- and/or poly-brominated products which have significantly lower pKa than that of the unsubstituted H-type structure, so that it can be used to extract lithium from a weakly alkaline aqueous solution. Alternatively, the preferred compound of Formula (I) containing a C₆₋₁₆ alkoxy chain can react with a dilute aqueous nitric acid solution at lower temperature to obtain mono- and/or poly-nitro-substituted products, or a mixture thereof, which also have significantly lower pKa than that of the unsubstituted H-type structure so that can be used to extract lithium from a weakly alkaline aqueous solution, too. The lower temperature is 10 to 70°C.

In a preferred embodiment, R2 in the compound of Formula (I) is H, nitro, or methoxy.

In a preferred embodiment, R3 in the compound of Formula (I) is H, nitro, methoxy, halogen, or benzyloxy.

In a preferred embodiment, R4 in the compound of Formula (I) is H, nitro, C₁₋₁₆ alkoxy, or benzyloxy, preferably C₆₋₁₆ alkoxy.

In a preferred embodiment, R5 in the compound of Formula (I) is H, nitro, or methoxy.

In a preferred embodiment, in the compound of Formula (I):
R1 is methyl, ethyl, propyl, or phenyl; and/or,
R2 is hydrogen, nitro, or methoxy; and/or,
R3 is hydrogen, halogen, methoxy, or nitro; and/or,
R4 is C₁₋₁₆ alkoxy, preferably C₆₋₁₆ alkoxy; and/or,
R5 is hydrogen, methoxy, and nitro.

The present invention further provides a liquid organic mixture for extracting lithium by means of extraction, comprising: one or more compounds of Formula (I) as described, a synergist, and optionally a diluent. Herein, the optional diluent indicates that a diluent may or may not be present in the liquid organic mixture for extracting lithium by means of extraction.

In a preferred embodiment, the liquid organic mixture for extracting lithium by means of extraction comprises one or more compounds of Formula (I), a synergist, and a diluent.

In an embodiment, the liquid organic mixture for extracting lithium by means of extraction consists of one or more compounds of Formula (I), a synergist, and a diluent.

The compound of Formula (I), taken together with the synergist and the diluent, constitutes a liquid organic mixture for extracting lithium from an aqueous lithium-containing solution, i.e., an extraction composition. The synergist is, e.g., neutral phosphine oxides, specially, one or more of tributyl phosphate, trioctyl phosphate, tris(2-ethylhexyl) phosphate, trihexyl phosphate, tripentyl phosphate, butyl dibutyl phosphate, dibutyl butyl phosphate, methyl di-sec-octyl phosphate, methyl di-isooctyl phosphate, isopropyl di-isooctyl phosphate, methyl di-isopentyl phosphate, triphenyl phosphine oxide, diphenyl benzyl phosphine oxide, diphenyl(2-hydroxybenzyl) phosphine oxide, 2,5-dihydroxyphenyl(diphenyl) phosphine oxide, trioctyl phosphine oxide (or Cyanex921), trialkyl phosphine oxide TRPO (or Cyanex923). The diluent is one or more of kerosene, D80 solvent oil, D60 solvent oil, heptane, cyclohexane, octane, dodecane, petroleum ether, xylene, anisole, methyl isobutyl ketone toluene, octanone 5-nonanone isopentanol, butanol, halobenzene, etc.

On the one hand, the addition of the synergist can enhance the acidity by forming hydrogen bonding with the compound of Formula (I); and on the other hand, it can also enhance the chemical stability of the resulting extraction composition under the action of alkaline solutions and air. The stronger the alkalinity of the synergist, the more significant the above effects. Thus, the preferred neutral phosphorus oxide synergists are tributyl phosphine oxide, trioctyl phosphine oxide, Cyanex923, and triphenyl phosphine oxide.

The aforementioned liquid organic mixture for extracting lithium by means of extraction can be used to extract lithium from an aqueous lithium-containing solution. The aqueous lithium-containing solution is neutral or alkaline brines with low magnesium and calcium content, geothermal brines, or lithium carbonate or lithium phosphate mother liquors. In the operation of extracting lithium by means of extraction, the compound of Formula (I) or a mixture thereof has a total concentration of 0.03 mol/L to 1.0 mol/L in the extracting agent, preferably 0.05 to 0.2 mol/L; the synergist has a concentration of 0.01 mol/L to 2.0 mol/L in the extracting agent, preferably 0.05 to 0.2 mol/L; and the molar ratio of the compound of Formula (I) to the synergist is 3:1 to 0.5:1, preferably 1.5:1 to 0.8:1. The aqueous solution containing lithium ions has a pH of 5 to 13, preferably 8 to 12.5. For the alkaline natural brines, they can directly interact with the organic solution containing an extracting agent so that the lithium ions from the brine enter the organic solution containing the extracting agent. For the aqueous lithium-containing solution with pH<7, the pH and alkalinity can be adjusted to 8 to 12.5 by adding a hydroxide, carbonate, phosphate, or borate of alkali metal or ammonium. Alternatively, an extracting agent saponified with sodium and/or potassium ions can directly interact with the aqueous lithium-containing solution with pH<7, so that the lithium ions in the brine can enter the organic solution containing an extracting agent via the ion exchange between the sodium and/or potassium ions and the lithium ions.

The alkali metal hydroxide or ammonium hydroxide used to adjust the pH and alkalinity of the aqueous lithium-containing solution or to saponify the extracting agent is one or more of sodium hydroxide, potassium hydroxide, and ammonium hydroxide; and/or the carbonate is one or more of sodium carbonate and potassium carbonate; and/or the phosphate is one or more of trisodium phosphate, tripotassium phosphate, triammonium phosphate, disodium hydrogen phosphate, dipotassium hydrogen phosphate, and diammonium hydrogen phosphate; and/or the borate is one or more of sodium metaborate, potassium metaborate, ammonium metaborate, sodium tetraborate, potassium tetraborate, and ammonium tetraborate.

In the operation of lithium ion extraction, the volume ratio of the aqueous lithium-containing solution to the extracting agent is 30:1 to 1:30, preferably 20:1 to 1:5; the aqueous lithium-containing solution has a lithium concentration of 0.01 mol/L to 2.0 mol/L, preferably a lithium concentration of 0.02 mol/L to 0.5 mol/L; and the extraction operation temperature is - 5°C to 40°C, preferably 15°C to 30°C.

The extraction process reaches equilibrium over 3 to 30 minutes, preferably 5 to 10 minutes. Subsequently, the mixture is subjected to liquid-liquid phase separation by settling or centrifugation to remove the remaining aqueous solution after extraction, and obtain an organic solution containing an extracting agent which has lithium ions loaded.

Furthermore, a stripping agent is used to interact with the extracting agent having lithium ions loaded to allow the lithium ions to enter the stripping agent so that the organic mixture regains its ability to extract lithium from an aqueous lithium-containing solution. The stripping agent is an aqueous solution of acid, preferably, one or more of carbonic acid, sulfuric acid, sulfurous acid, hydrochloric acid, nitric acid, and phosphoric acid. In the stripping agent, the concentration of hydrogen ions is 0.05 mol/L to 6 mol/L, preferably 0.5 mol/L to 3 mol/L. If the hydrogen ion concentration in the stripping agent is too low, it is not conducive to the enrichment of lithium ions; and if the hydrogen ion concentrate in the stripping agent is too high, it can lead to the entrainment of a large amount of acid in the regenerated extracting agent, which will affect the subsequent extraction. The volume ratio of the stripping agent to the extracting agent having lithium ions loaded is 1:1 to 1:50, preferably 1:3 to 1:20. After reaching the stripping equilibrium, the lithium concentration in the stripping agent is 0.05 mol/L to 10 mol/L, preferably 0.5 mol/L to 5 mol/L. The interaction time between the extracting agent having lithium ions loaded and the stripping agent is 3 to 30 minutes, preferably 5 to 10 minutes.

The interaction between the extracting agent and the aqueous lithium-containing solution and/or the lithium ion stripping agent is carried out in a mixer-settler, centrifugal extractor, or extraction column, and the lithium extraction, separation of lithium from sodium or potassium, and lithium concentration can be achieved by multi-stage extraction, washing, and stripping.

To increase the concentration of lithium ions in the stripping agent, reduce the equipment investment for subsequent concentration, and lower the production costs, the stripping agent containing lithium can be repeatedly supplemented with concentrated acid and then reused as a stripping agent to interact with the extracting agent having lithium ions loaded, thereby achieving multiple enrichments of lithium ions in the stripping agent. Preferred concentrated acid is 6~12 mol/L of hydrochloric acid, 6~18 mol/L of sulfuric acid, 6~14 mol/L of nitric acid, or 6~15 mol/L of phosphoric acid.

Further, the operation of lithium concentration is performed by evaporating water from the lithium-containing stripping agent, and then sodium carbonate, ammonium carbonate, etc. are added to precipitate lithium carbonate. Also, the mother liquor remaining upon precipitation of lithium carbonate can be reused with the extracting agent as described in the present invention for lithium extraction.

The present invention includes, but is not limited to, the following embodiments:
1. Compound of Formula (I) as lithium extracting agent, 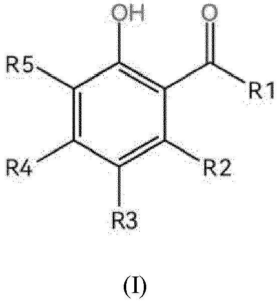
   wherein R1 is H, C₁₋₈ alkyl, C₃₋₆ cycloalkyl, or phenyl, wherein the C₁₋₈ alkyl, C₃₋₆ cycloalkyl, or phenyl is optionally substituted with one or more substituents selected from the group consisting of hydroxyl, halogen, C₁₋₆ alkyl, C₁₋₆ alkoxy, or nitro, and the C₁₋₆ alkyl, C₁₋₆ alkoxy is optionally further substituted with one or more substituents selected from the group consisting of hydroxyl, halogen, C₁₋₆ alkyl, C₁₋₆ alkoxy, or nitro; and
   R2, R3, R4 and R5 are each independently hydrogen, halogen, nitro, benzyloxy, C₁₋₆ alkyl, or C₁₋₁₆ alkoxy, where the benzyloxy, C₁₋₆ alkyl, or C₁₋₁₆ alkoxy is optionally substituted with one or more substituents selected from the group consisting of hydroxyl, halogen, C₁₋₆ alkyl, C₁₋₆ alkoxy, and nitro.
2 . The compound of Formula (I) according to Embodiment 1, wherein R1 is C₁₋₆ alkyl or phenyl, where the C₁₋₆ alkyl, or phenyl is optionally substituted with one or more substituents selected from the group consisting of hydroxyl, halogen, C₁₋₆ alkyl, phenyl, or C₁₋₆ alkoxy.
3. The compound of Formula (I) according to Embodiment 1 or 2, wherein at least one of R2, R3, R4 and R5 is nitro.
4. The compound of Formula (I) according to any one of Embodiments 1-3, wherein at least one of R2, R3, R4 and R5 is benzyloxy or unsubstituted or halogen substituted C₁₋₁₆ alkoxy, e.g., R4 is benzyloxy or unsubstituted or halogen substituted C₁₋₁₆ alkoxy.
5. The compound of Formula (I) according to Embodiment 4, wherein at least one of R2, R3, R4 and R5 is unsubstituted or halogen substituted C₆₋₁₆ alkoxy, preferably, R4 is unsubstituted or halogen substituted C₆₋₁₆ alkoxy.
6. The compound of Formula (I) according to any one of Embodiments 1-5, wherein at least one of R2, R3, R4 and R5 is halogen.
7. The compound of Formula (I) according to any one of Embodiments 1-6, wherein R2 is H, nitro, or methoxy.
8. The compound of Formula (I) according to any one of Embodiments 1-7, wherein R3 is H, nitro, methoxy, halogen, or benzyloxy.
9. The compound of Formula (I) according to any one of Embodiments 1-8, wherein R4 is H, nitro, C₁₋₁₆ alkoxy, or benzyloxy.
10. The compound of Formula (I) according to any one of Embodiments 1-9, wherein R5 is H, nitro, or methoxy.
11. The compound of Formula (I) according to any one of Embodiments 1-10, wherein
   R1 is methyl, ethyl, propyl, or phenyl; and/or
   R2 is hydrogen, nitro, or methoxy; and/or
   R3 is hydrogen, halogen, methoxy, or nitro; and/or
   R4 is C₁₋₁₆ alkoxy; and/or
   R5 is hydrogen, methoxy, and nitro.
12. The compound of Formula (I) according to Embodiment 1, selected from: 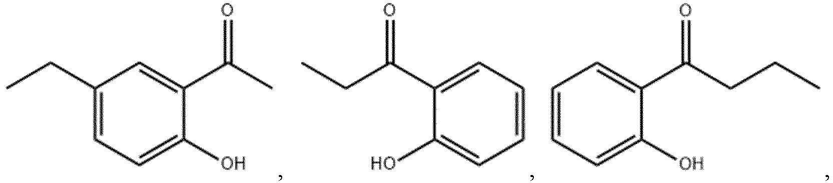 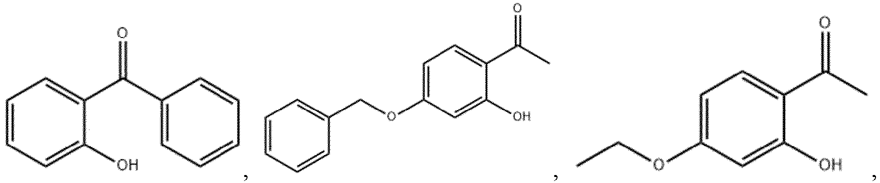 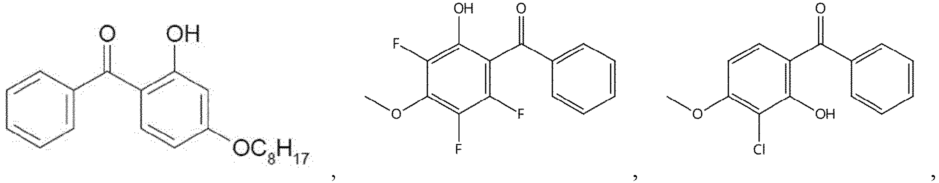 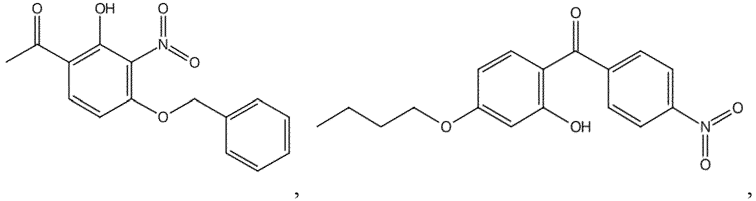 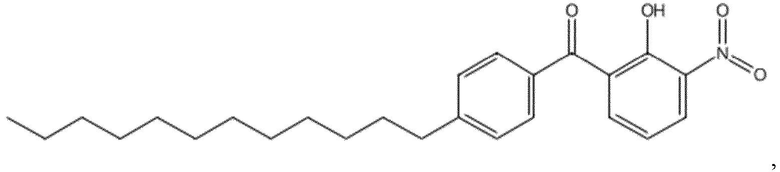 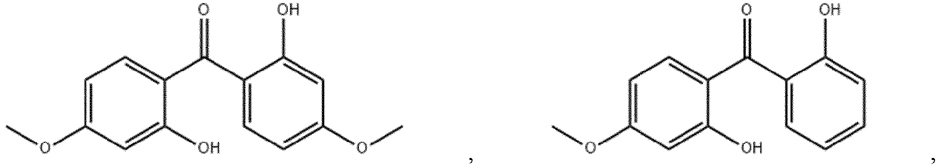 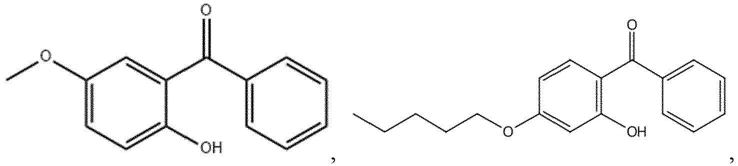 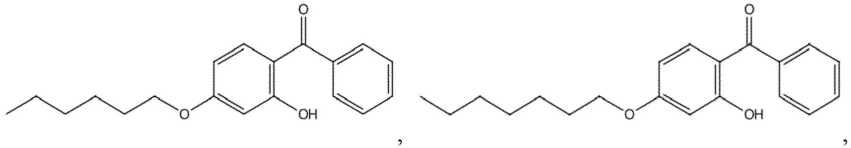 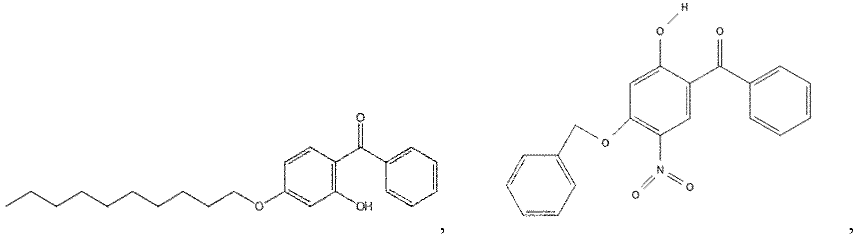 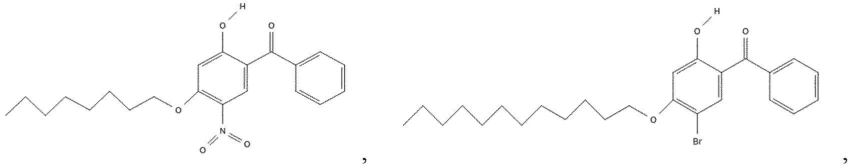 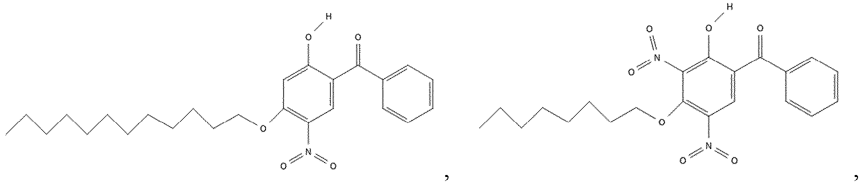 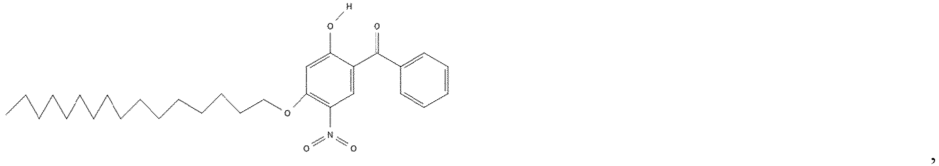 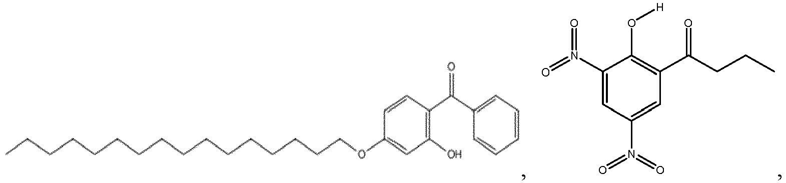 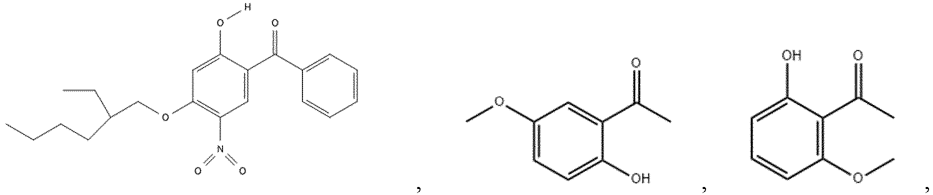 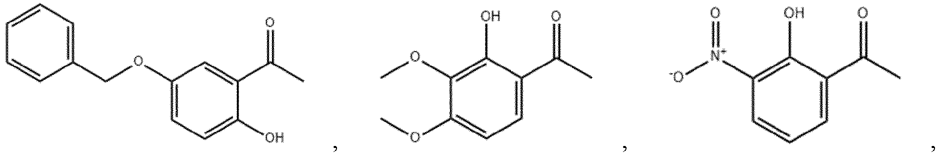 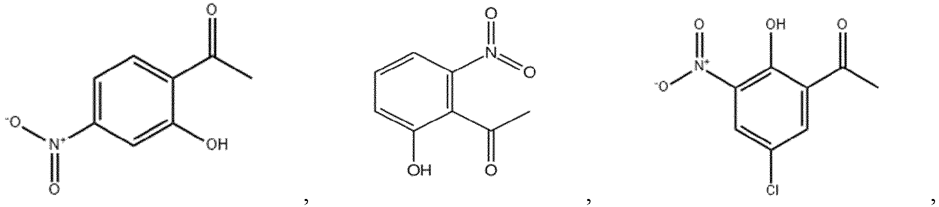 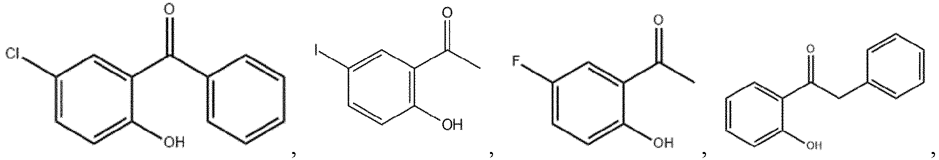 and 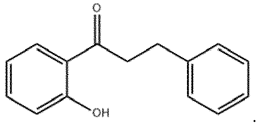
13. Use of the compound of Formula (I) according to any one of Embodiments 1-12 in lithium extraction as lithium extracting agent.
14. A liquid organic mixture for extracting lithium by means of extraction, comprising:
   one or more of the compound of Formula (I) according to any of Embodiments 1-12,
   a synergist, and
   optionally a diluent.
15. The liquid organic mixture for extracting lithium by means of extraction according to Embodiment 14, wherein
   the synergist is one or more of tributyl phosphate, trioctyl phosphate, tris(2-ethylhexyl) phosphate, trihexyl phosphate, tripentyl phosphate, butyl dibutyl phosphate, dibutyl butyl phosphate, methyl di-sec-octyl phosphate, methyl di-isooctyl phosphate, isopropyl di-isooctyl phosphate, methyl di-isopentyl phosphate, triphenyl phosphine oxide, diphenyl benzyl phosphine oxide, diphenyl(2-hydroxybenzyl) phosphine oxide, 2,5-dihydroxyphenyl(diphenyl) phosphine oxide, trioctyl phosphine oxide (or Cyanex921), trialkyl phosphine oxide TRPO (or Cyanex923).
16. The liquid organic mixture for extracting lithium by means of extraction according to Embodiment 14 or 15, wherein
   the diluent is one or more of kerosene, D80 solvent oil, D60 solvent oil, heptane, cyclohexane, octane, dodecane, petroleum ether, xylene, anisole, methyl isobutyl ketone toluene, octanone 5-nonanone isopentanol, butanol, and halobenzene.
17. The liquid organic mixture for extracting lithium by means of extraction according to any one of Embodiments 14-16, wherein the compound of Formula (I) has a total concentration of 0.03 mol/L to 1.0 mol/L in the liquid organic mixture for extracting lithium by means of extraction; the synergist has a concentration of 0.01 mol/L to 2.0 mol/L in the liquid organic mixture for extracting lithium by means of extraction; and the molar ratio of the compound of Formula (I) to the synergist is 3:1 to 0.5:1.
18. A method for extracting lithium by means of extraction, comprising:
   contacting the liquid organic mixture for extracting lithium by means of extraction according to any one of Embodiments 14-17 with an aqueous solution containing lithium ions, allowing the lithium ions from the aqueous solution to enter an organic layer by liquid-liquid phase separation, and collecting the organic layer.
19. The method according to Embodiment 18, wherein the aqueous solution containing lithium ions has a pH of 7~13 before contact with the liquid organic mixture for extracting lithium by means of extraction.
20. The method according to Embodiment 19, wherein the alkalinity of the aqueous solution containing lithium ions is adjusted by adding a hydroxide, a carbonate, a phosphate, or a borate of alkali metal or ammonium.
21. The method according to Embodiment 19, wherein the aqueous solution containing lithium ions is a naturally occurring brine with alkalinity; and optionally the alkalinity of the naturally occurring brine with alkalinity is further adjusted by adding a hydroxide, a carbonate, a phosphate, or a borate of alkali metal or ammonium.
22. The method according to any one of Embodiments 18 to 20, wherein the aqueous solution containing lithium ions is a mother liquor from production of lithium carbonate and/or a mother liquor from production of lithium phosphate.
23. The method according to Embodiment 20, wherein the hydroxide of alkali metal or ammonium is one or more of sodium hydroxide, potassium hydroxide, and ammonium hydroxide; the carbonate is one or more of sodium carbonate and potassium carbonate; the phosphate is one or more of trisodium phosphate, tripotassium phosphate, triammonium phosphate, disodium hydrogen phosphate, dipotassium hydrogen phosphate, and diammonium hydrogen phosphate; and the borate is one or more of sodium metaborate, potassium metaborate, ammonium metaborate, sodium tetraborate, potassium tetraborate, and ammonium tetraborate.
24. The method according to Embodiment 18, wherein the aqueous solution containing lithium ions has a pH of 5~8 before contact with the liquid organic mixture for extracting lithium by means of extraction; the liquid organic mixture for extracting lithium by means of extraction is previously saponified with one or more of sodium hydroxide, potassium hydroxide, sodium carbonate, potassium carbonate, sodium bicarbonate, or potassium bicarbonate solution before contact with the aqueous solution containing lithium ions.
25. The method according to any one of Embodiments 18 to 24, wherein the volume ratio of the aqueous solution containing lithium ions to the liquid organic mixture for extracting lithium by means of extraction is 30:1 to 1:30; and/or the aqueous solution containing lithium ions has a lithium concentration of 0.01 mol/L to 2.0 mol/L.
26. The method according to any one of Embodiments 18 to 25, further comprising a step of contacting the stripping agent with the organic layer to obtain a lithium-containing stripping agent.
27. The method according to Embodiment 26, wherein the stripping agent is an aqueous solution of acid.
28. The method according to Embodiment 27, wherein the stripping agent is an aqueous solution of one or more of carbonic acid, sulfuric acid, sulfurous acid, hydrochloric acid, nitric acid, and phosphoric acid; and/or, in the stripping agent, the concentration of hydrogen ions is 0.05 mol/L to 6 mol/L; and the volume ratio of the stripping agent to the organic layer is 1:1 to 1:50.
29. The method according to Embodiment 26, wherein the stripping agent has a lithium concentration of 0.05 mol/L to 10 mol/L after contact with the organic layer.
30. The method according to any one of Embodiments 18 to 29, wherein the contact time of the liquid organic mixture for extracting lithium by means of extraction and the aqueous solution containing lithium ions is 3 to 30 minutes.
31. The method according to any one of Embodiments 26 to 30, wherein the contact time of the stripping agent and the organic layer is 3 to 30 minutes.
32. The method according to any one of Embodiments 18 to 31, wherein the contact is carried out in a mixer-settler, centrifugal extractor, or extraction column.
33. The method according to any one of Embodiments 18 to 32, wherein the liquid organic mixture for extracting lithium by means of extraction is contacted with the same aqueous solution containing lithium ions one or more times.
34. The method according to any one of Embodiments 26 to 33, wherein the lithium-containing stripping agent is supplemented with a concentrated acid after contact with the organic layer, and then reused as a stripping agent to contact the organic layer; where the concentrated acid is 6~12 mol/L of hydrochloric acid, 6~18 mol/L of sulfuric acid, 6~14 mol/L of nitric acid, or 6~15 mol/L of phosphoric acid.
35. The method according to any one of Embodiments 26 to 34, further comprising an operation of concentrating lithium by evaporating water from the lithium-containing stripping agent, and/or further precipitating lithium carbonate from the lithium-containing stripping agent.

### EXAMPLES

The following examples are provided to further illustrate the present invention. It should be understood that these examples are for illustrative purposes only and are not intended to limit the scope of the invention. Where specific techniques, or conditions are not specified in the examples, they should be carried out according to the techniques, or conditions described in the literature of the field, or according to the product instructions. Reagents, or instruments not specified with a manufacturer are all conventional products that can be obtained through commercial purchase.

### Example 1

Preparation of Extracting agent Organic Phase: 1 mmol of 2-hydroxydibenzophenone (with the structure of 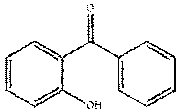 ) and 1 mmol of trioctyl phosphine oxide were added to 10 mL of cyclohexane, and placed in a separatory funnel, and shaken until the solids were fully dissolved to form the extracting agent organic phase. Then, 4 mL of aqueous lithium-containing solution of B1 was added, shaken at 28°C for 10 minutes, and then subjected to settlement and phase separation. The remaining aqueous phase R1 at the bottom was drained. 0.2 mL was sampled, diluted to 50 mL, and detected for the concentrations of components using an Inductively Coupled Plasma Optical Emission Spectrometer (ICP) (the same detection method is also used in the subsequent examples). The results are shown in the table below.

| | c_{Li}/(g/L) | c_{Na}/(g/L) | c_{K}/(g/L) | pH |
|---|---|---|---|---|
| Aqueous lithium-containing solution B1 | 0.807 | 56.12 | None | 11.48 |
| Remaining aqueous phase R1 | 0.257 | 50.50 | None | 10.12 |

The extraction rates for lithium and sodium ions (*E* = (c_{B1} - c_{R1})c_{B1} × 100%, the same below) are 68.2% and 10%, respectively. The separation factor of Li/Na (*β*_{Li/Na} = (c_{Li}/c_{Na})_{B1-R1}/(c_{Li}/c_{Na})_{R1}, throughout the application) is 19.2.

### Example 2

Preparation of Extracting agent Organic Phase: 1 mmol of 2-hydroxydibenzophenone (with the structure of 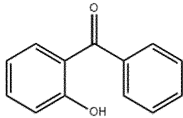 ) and 1 mmol of tributyl phosphate were added to 10 mL of cyclohexane, and placed in a separatory funnel, and shaken until the solids were fully dissolved to form the extracting agent organic phase. Then, 4 mL of aqueous lithium-containing solution of B1 was added, shaken at 18°C for 10 minutes, and then subjected to settlement and phase separation. The remaining aqueous phase R1 at the bottom was drained. The results are shown in the table below.

| | c_{Li}/(g/L) | c_{Na}/(g/L) | c_{K}/(g/L) | pH |
|---|---|---|---|---|
| Aqueous lithium-containing solution B1 | 0.807 | 56.12 | None | 11.48 |
| Remaining aqueous phase R1 | 0.362 | 50.23 | None | - |

The extraction rates for lithium and sodium ions are 55.1% and 10.5%, respectively. The separation factor of Li/Na is 10.5.

### Example 3

Preparation of Extracting agent Organic Phase: 0.5 mmol of 2-hydroxypropiophenone (with the structure of 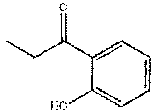 ) and 1 mmol of tributyl phosphine oxide were added to 10 mL of kerosene, and placed in a separatory funnel to form the extracting agent organic phase. Then, 4 mL of aqueous lithium-containing solution of B1 was added, shaken for 10 minutes, and then subjected to settlement and phase separation. The remaining aqueous phase R1 at the bottom was drained. The analysis results are shown in the table below.

| | c_{Li}/(g/L) | c_{Na}/(g/L) | c_{K}/(g/L) | pH |
|---|---|---|---|---|
| Aqueous lithium-containing solution B1 | 0.807 | 56.12 | None | 11.48 |
| Remaining aqueous phase R1 | 0.383 | 52.10 | None | - |

The extraction rates for lithium and sodium ions are 52.5% and 7.2%, respectively. The separation factor of Li/Na is 14.3.

### Example 4

Preparation of Extracting agent Organic Phase: 0.5 mmol of 2-hydroxybutyrophenone (with the structure of 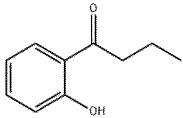 ) and 0.5 mmol of trioctyl phosphine oxide were added to 10 mL of kerosene, and placed in a separatory funnel to form the extracting agent organic phase. Then, 4 mL of aqueous lithium-containing solution of B1 was added, shaken for 5 minutes, and then subjected to settlement and phase separation. The remaining aqueous phase R1 at the bottom was drained. The results are shown in the table below.

| | c_{Li}/(g/L) | c_{Na}/(g/L) | c_{K}/(g/L) | pH |
|---|---|---|---|---|
| Aqueous lithium-containing solution B1 | 0.807 | 56.12 | None | 11.48 |
| Remaining aqueous phase R1 of Example 1 | 0.322 | 53.42 | None | - |

The extraction rates for lithium and sodium ions are 60.1% and 4.8%, respectively. The separation factor of Li/Na is 29.8.

### Example 5

Preparation of Extracting agent Organic Phase: 1 mmol of 2-hydroxybutyrophenone and 2 mmol of tributyl phosphate were added to 10 mL of kerosene, and placed in a separatory funnel to form the extracting agent organic phase. Then, 4 mL of aqueous lithium-containing solution of B1 was added, shaken for 5 minutes, and then subjected to settlement and phase separation. The remaining aqueous phase R1 at the bottom was drained. The analysis results are shown in the table below.

| | c_{Li}/(g/L) | c_{Na}/(g/L) | c_{K}/(g/L) | pH |
|---|---|---|---|---|
| Aqueous lithium-containing solution B1 | 0.807 | 56.12 | None | 11.48 |
| Remaining aqueous phase R1 | 0.157 | 52.32 | None | - |

The extraction rates for lithium and sodium ions are 80.5% and 6.8%, respectively. The separation factor of Li/Na is 57.0.

### Example 6

Preparation of Extracting agent Organic Phase: 2 mmol of 4-benzyloxy-2-hydroxyacetophenone (with the structure of 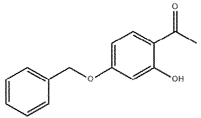 ) and 4 mmol of trioctyl phosphine oxide were added to 20 mL of kerosene, and placed in a separatory funnel to form the extracting agent organic phase. Then, 4 mL of aqueous lithium-containing solution of B1 was added, shaken for 3 minutes, and then subjected to settlement and phase separation. The remaining aqueous phase R1 at the bottom was drained. The analysis results are shown in the table below.

| | c_{Li}/(g/L) | c_{Na}/(g/L) | c_{K}/(g/L) | pH |
|---|---|---|---|---|
| Aqueous lithium-containing solution B1 | 0.807 | 56.12 | None | 11.48 |
| Remaining aqueous phase R1 | 0.527 | 55.34 | None | - |

The extraction rates for lithium and sodium ions are 34.7% and 1.4%, respectively. The separation factor of Li/Na is 37.7.

### Example 7

Preparation of Extracting agent Organic Phase: 1.5 mmol of 4-pentyl-2-hydroxydibenzophenone (with the structure of 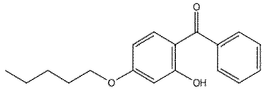 ) and 1.4 mmol of trioctyl phosphine oxide were added to 10 mL of cyclohexane, and placed in a separatory funnel to form the extracting agent organic phase. Then, 4 mL of aqueous lithium-containing solution of B1 was added, shaken for 10 minutes, and then subjected to settlement and phase separation. The remaining aqueous phase R1 at the bottom was drained. The analysis results are shown in the table below.

| | c_{Li}/(g/L) | c_{Na}/(g/L) | c_{K}/(g/L) | pH |
|---|---|---|---|---|
| Aqueous lithium-containing solution B1 | 0.807 | 56.12 | None | 11.48 |
| Remaining aqueous phase R1 | 0.186 | 51.53 | None | - |

The extraction rates for lithium and sodium ions are 77.0% and 8.2%, respectively. The separation factor of Li/Na is 37.5.

### Example 8

Preparation of Extracting agent Organic Phase: 1 mmol of 4-hyxyloxy-2-hydroxydibenzophenone (with the structure of 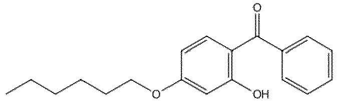 ) and 1 mmol of trioctyl phosphine oxide were added to 10 mL of D80 solvent oil, and placed in a separatory funnel to form the extracting agent organic phase. Then, 4 mL of aqueous lithium-containing solution of B1 was added, shaken for 10 minutes, and then subjected to settlement and phase separation. The remaining aqueous phase R1 at the bottom was drained. The analysis results are shown in the table below.

| | c_{Li}/(g/L) | c_{Na}/(g/L) | c_{K}/(g/L) | pH |
|---|---|---|---|---|
| Aqueous lithium-containing solution B1 | 0.807 | 56.12 | None | 11.48 |
| Remaining aqueous phase R1 | 0.266 | 52.50 | None | - |

The extraction rates for lithium and sodium ions are 67.0% and 6.5%, respectively. The separation factor of Li/Na is 29.5.

### Example 9

Preparation of Extracting agent Organic Phase: 1 mmol of 4-heptoxy-2-hydroxydibenzophenone (with the structure of 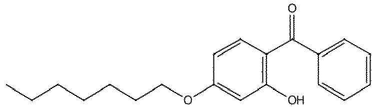 ) and 1 mmol of tri(2-ethylhexyl) phosphate were added to 10 mL of D80 solvent oil, and placed in a separatory funnel to form the extracting agent organic phase. Then, 4 mL of aqueous lithium-containing solution of B1 was added, shaken for 10 minutes, and then subjected to settlement and phase separation. The remaining aqueous phase R1 at the bottom was drained. The analysis results are shown in the table below.

| | c_{Li}/(g/L) | c_{Na}/(g/L) | c_{K}/(g/L) | pH |
|---|---|---|---|---|
| Aqueous lithium-containing solution B1 | 0.807 | 56.12 | None | 11.48 |
| Remaining aqueous phase R1 | 0.316 | 52.65 | None | - |

The extraction rates for lithium and sodium ions are 60.8% and 6.2%, respectively. The separation factor of Li/Na is 23.6.

### Example 10

Preparation of Extracting agent Organic Phase: 1.5 mmol of 4-octyloxy-2-hydroxydibenzophenone (with the structure of 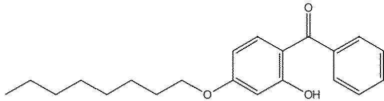 ) and 1.5 mmol of trioctyl phosphine oxide were added to 10 mL of cyclohexane, and placed in a separatory funnel to form the extracting agent organic phase. Then, 4 mL of aqueous lithium-containing solution of B2 was added, shaken for 10 minutes, and then subjected to settlement and phase separation. The remaining aqueous phase R1 at the bottom was drained. The analysis results are shown in the table below.

| | c_{Li}/(g/L) | c_{Na}/(g/L) | c_{K}/(g/L) | pH |
|---|---|---|---|---|
| Aqueous lithium-containing solution B2 | 0.947 | 51.63 | None | 10.77 |
| Remaining aqueous phase R1 | 0.854 | 49.31 | None | - |

The extraction rates for lithium and sodium ions are 9.8% and 4.5%, respectively. The separation factor of Li/Na is 2.3.

### Example 11

Preparation of Extracting agent Organic Phase: 1.5 mmol of 4-decyloxy-2-hydroxydibenzophenone (with the structure of 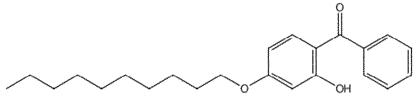 ) and 1.5 mmol of trioctyl phosphine oxide were added to 10 mL of cyclohexane, and placed in a separatory funnel to form the extracting agent organic phase. Then, 4 mL of aqueous lithium-containing solution of B3 was added, shaken for 10 minutes, and then subjected to settlement and phase separation. The remaining aqueous phase R1 at the bottom was drained. The analysis results are shown in the table below.

| | c_{Li}/(g/L) | c_{Na}/(g/L) | c_{K}/(g/L) | pH |
|---|---|---|---|---|
| Aqueous lithium-containing solution B3 | 0.937 | 119.10 | 43.03 | 9.60 |
| Remaining aqueous phase R1 | 0.908 | 118.01 | 42.65 | - |

The extraction rates for lithium and sodium ions are 3.1% and 0.9%, respectively. The separation factor of Li/Na is 3.5.

### Example 12

Preparation of Extracting agent Organic Phase: 2 mmol of 4-benzyloxy-5-nitro-2-hydroxydibenzophenone (with the structure of 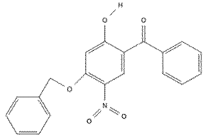 ) and 2 mmol of trioctyl phosphine oxide were added to 20 mL of n-heptane, and placed in a separatory funnel to form the extracting agent organic phase. Then, 4 mL of aqueous lithium-containing solution of B3 was added, shaken for 10 minutes, and then subjected to settlement and phase separation. The remaining aqueous phase R1 at the bottom was drained. The analysis results are shown in the table below.

| | c_{Li}/(g/L) | c_{Na}/(g/L) | c_{K}/(g/L) | pH |
|---|---|---|---|---|
| Aqueous lithium-containing solution B3 | 0.937 | 119.10 | 43.03 | 9.60 |
| Remaining aqueous phase R1 | 0.134 | 107.08 | 39.45 | 8.66 |

The extraction rates for lithium, sodium and potassium ions are 85.7%, 10.1% and 8.3%, respectively. The separation factors of Li/Na and Li/K are 53.4 and 66.0, respectively.

### Example 13

Preparation of Extracting agent Organic Phase: 1 mmol of 4-octyloxy-5-nitro-2-hydroxydibenzophenone (with the structure of 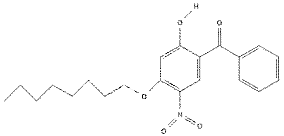 ) and 1 mmol of trioctyl phosphine oxide were added to 10 mL of n-heptane, and placed in a separatory funnel to form the extracting agent organic phase. Then, 4 mL of aqueous lithium-containing solution of B3 was added, shaken for 10 minutes, and then subjected to settlement and phase separation. The remaining aqueous phase R1 at the bottom was drained. The analysis results are shown in the table below.

| | c_{Li}/(g/L) | c_{Na}/(g/L) | c_{K}/(g/L) | pH |
|---|---|---|---|---|
| Aqueous lithium-containing solution B3 | 0.937 | 119.10 | 43.03 | 9.60 |
| Remaining aqueous phase R1 | 0.331 | 112.87 | 41.14 | - |

The extraction rates for lithium, sodium and potassium ions are 64.7%, 5.2% and 4.4%, respectively. The separation factors of Li/Na and Li/K are 33.2 and 39.9, respectively.

### Example 14

Preparation of Extracting agent Organic Phase: 2 mmol of 4-dodecyloxy-5-bromo-2-hydroxydibenzophenone (with the structure of 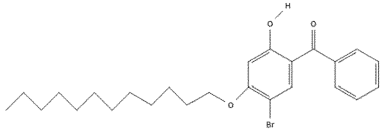 ) and 4 mmol of trioctyl phosphine oxide were added to 20 mL of n-heptane, and placed in a separatory funnel to form the extracting agent organic phase. Then, 10 mL of aqueous lithium-containing solution of B4 was added, shaken for 10 minutes, and then subjected to settlement and phase separation. The remaining aqueous phase R1 at the bottom was drained. The analysis results are shown in the table below.

| | c_{Li}/(g/L) | c_{Na}/(g/L) | c_{K}/(g/L) | pH |
|---|---|---|---|---|
| Aqueous lithium-containing solution B4 | 0.152 | 34.20 | 2.36 | 9.14 |
| Remaining aqueous phase R1 | 0.078 | 32.20 | 1.77 | - |

The extraction rates for lithium, sodium and potassium ions are 48.7%, 5.8% and 25.0%, respectively. The separation factors of Li/Na and Li/K are 15.3 and 2.8, respectively.

### Example 15

Preparation of Extracting agent Organic Phase: 2 mmol of 4-dodecyloxy-5-bromo-2-hydroxydibenzophenone (with the structure of 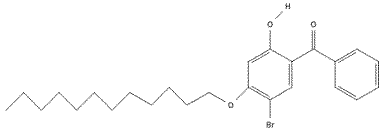 ) and 2 mmol of trioctyl phosphine oxide were added to 20 mL of n-heptane, and placed in a separatory funnel to form the extracting agent organic phase. Then, 5 mL of aqueous lithium-containing solution of B4 was added, shaken for 10 minutes, and then subjected to settlement and phase separation. The remaining aqueous phase R1 at the bottom was drained. The analysis results are shown in the table below.

| | c_{Li}/(g/L) | c_{Na}/(g/L) | c_{K}/(g/L) | pH |
|---|---|---|---|---|
| Aqueous lithium-containing solution B4 | 0.152 | 34.20 | 2.36 | 9.14 |
| Remaining aqueous phase R1 | 0.054 | 31.83 | 1.80 | - |

The extraction rates for lithium, sodium and potassium ions are 64.5%, 6.9% and 23.7%, respectively. The separation factors of Li/Na and Li/K are 24.4 and 5.8, respectively.

### Example 16

Preparation of Extracting agent Organic Phase: 2 mmol of 4-octyloxy-2,5-dinitro-2-hydroxydibenzophenone (with the structure of 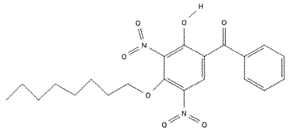 ) and 2 mmol of trioctyl phosphine oxide were added to 20 mL of cyclohexane, and placed in a separatory funnel to form the extracting agent organic phase. Then, 4 mL of aqueous lithium-containing solution of B5 was added, shaken for 10 minutes, and then subjected to settlement and phase separation. The remaining aqueous phase R1 at the bottom was drained. The analysis results are shown in the table below.

| | c_{Li}/(g/L) | c_{Na}/(g/L) | c_{K}/(g/L) | pH |
|---|---|---|---|---|
| Aqueous lithium-containing solution B5 | 0.261 | 39.98 | 7.61 | 9.49 |
| Remaining aqueous phase R1 | 0.030 | 34.90 | 6.89 | - |

The extraction rates for lithium, sodium and potassium ions are 88.5%, 12.7% and 9.5%, respectively. The separation factors of Li/Na and Li/K are 52.9 and 73.7, respectively.

### Example 17

Preparation of Extracting agent Organic Phase: 1 mmol of 4-octyloxy-5-nitro-2-hydroxydibenzophenone (with the structure of 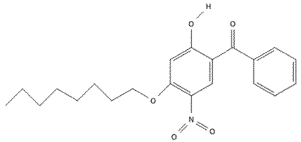 ), 1 mmol of 4-octyloxy-3,5-dinitro-2-hydroxydibenzophenone (with the structure of 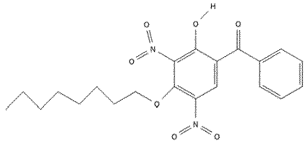 ) and 2 mmol of trioctyl phosphine oxide were added to 20 mL of kerosene, and placed in a separatory funnel to form the extracting agent organic phase. Then, 4 mL of aqueous lithium-containing solution of B5 was added, shaken for 10 minutes, and then subjected to settlement and phase separation. The remaining aqueous phase R1 at the bottom was drained. The analysis results are shown in the table below.

| | c_{Li}/(g/L) | c_{Na}/(g/L) | c_{K}/(g/L) | pH |
|---|---|---|---|---|
| Aqueous lithium-containing solution B5 | 0.261 | 39.98 | 7.61 | 9.49 |
| Remaining aqueous phase R1 | 0.035 | 34.67 | 6.25 | - |

The extraction rates for lithium, sodium and potassium ions are 86.6%, 13.3% and 17.9%, respectively. The separation factors of Li/Na and Li/K are 42.2 and 29.7, respectively.

### Example 18

Preparation of Extracting agent Organic Phase: 1.5 mmol of 4-hexadecyloxy-5-nitro-2-hydroxydibenzophenone (with the structure of 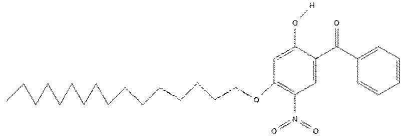 ), 0.5 mmol of 4-hexadecyloxy -2-hydroxydibenzophenone (with the structure of 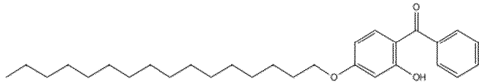 ) and 2 mmol of trioctyl phosphine oxide were added to 20 mL of kerosene, and placed in a separatory funnel to form the extracting agent organic phase. Then, 4 mL of aqueous lithium-containing solution of B5 was added, shaken for 10 minutes, and then subjected to settlement and phase separation. The remaining aqueous phase R1 at the bottom was drained. The analysis results are shown in the table below.

| | c_{Li}/(g/L) | c_{Na}/(g/L) | c_{K}/(g/L) | pH |
|---|---|---|---|---|
| Aqueous lithium-containing solution B5 | 0.261 | 39.98 | 7.61 | 9.49 |
| Remaining aqueous phase R1 | 0.066 | 35.03 | 6.72 | - |

The extraction rates for lithium, sodium and potassium ions are 74.7%, 12.4% and 11.7%, respectively. The separation factors of Li/Na and Li/K are 20.8 and 22.3, respectively.

### Example 19

The equilibrated aqueous solution from Example 14 was drained, leaving the organic mixture having lithium loaded. Then, 2 mL of 0.6 M sulfuric acid aqueous solution was added, shaken for 5 minutes, and then subjected to settlement and phase separation. The remaining aqueous phase FC1 at the bottom was drained. The analysis results are shown in the table below.

| | c_{Li}/(g/L) | c_{Na}/(g/L) | c_{K}/(g/L) | pH |
|---|---|---|---|---|
| Lithium-containing solution FC1 obtained from stripping | 0.332 | 3.33 | 0.16 | < 1 |

### Example 20

The equilibrated aqueous solution from Example 16 was drained, leaving the organic mixture having lithium loaded. Then, 2 mL of 0.6 M sulfuric acid aqueous solution was added, shaken for 3 minutes, and then subjected to settlement and phase separation. The remaining aqueous phase FC1 at the bottom was drained. The analysis results are shown in the table below.

| | c_{Li}/(g/L) | c_{Na}/(g/L) | c_{K}/(g/L) | pH |
|---|---|---|---|---|
| Lithium-containing solution FC1 obtained from stripping | 0.449 | 3.64 | 0.29 | < 1 |

### Example 21

Preparation of Extracting agent Organic Phase: 40 mmol of 2- hydroxy butyrophenone was dissolved in 80 mL of glacial acetic acid, and 3 mL of 68 wt.% concentrated nitric acid was added. The mixture was reacted at 40°C for 48 hours, then dried under reduced pressure at 70°C to obtain a solid powder product (mainly composed of 2-hydroxy-3,5-dinitro-butyrophenone with the structure of 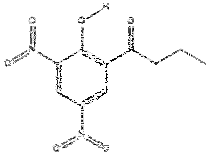 ). 2 mmol of the product and 2 mmol of trioctyl phosphine oxide were added together to 20 mL of D60 solvent oil, and placed in a separatory funnel to form the extracting agent organic phase. This organic phase was used to perform six extraction-stripping operations on the aqueous lithium-containing solution of B3. For each operation, 4 mL of the lithium-containing solution was added, shaken for 10 minutes, and then subjected to settlement and phase separation. The remaining aqueous phase R1 at the bottom to R6 were released. Afterward, 2 mL of water was added for washing, and the washing liquid was drained. Then, 2 mL of 0.6 M sulfuric acid aqueous solution was used to strip lithium (for the n^{th} stripping, the lithium-containing stripping solution from the n-1^{th} operation was used to continuously increase the lithium concentration in the stripping solution). The analysis results for each remaining aqueous phase and stripping solution (FC1 to FC6) are shown in the table below. The lithium ion concentrations in the six remaining aqueous phases were essentially consistent, all maintained at about 0.1 g/L, with a lithium extraction rate of approximately 90%, and the lithium concentration in the final stripping solution was 3.416 g/L.

| | c_{Li}/(g/L) | c_{Na}/(g/L) | c_{K}/(g/L) | pH |
|---|---|---|---|---|
| Lithium-containing solution B3 | 0.937 | 119.10 | 43.03 | 9.60 |
| Remaining aqueous phase R1 | 0.098 | 110.09 | 40.85 | - |
| Stripping aqueous phase FC1 | 0.800 | 3.56 | 0.45 | |
| Remaining aqueous phase R2 | 0.095 | 112.94 | 42.00 | |
| Stripping aqueous phase FC2 | 1.417 | 7.31 | 1.23 | |
| Remaining aqueous phase R3 | 0.099 | 112.25 | 41.74 | |
| Stripping aqueous phase FC3 | 2.046 | 9.52 | 1.48 | |
| Remaining aqueous phase R4 | 0.095 | 113.21 | 42.06 | |
| Stripping aqueous phase FC4 | 2.524 | 10.65 | 1.61 | |
| Remaining aqueous phase R5 | 0.106 | 111.12 | 41.42 | |
| Stripping aqueous phase FC5 | 2.959 | 11.13 | 1.64 | |
| Remaining aqueous phase R6 | 0.101 | 111.75 | 41.28 | |
| Stripping aqueous phase FC6 | 3.416 | 12.31 | 2.09 | |

### Example 22

Preparation of Extracting agent Organic Phase: 2 mmol of the synthesis product of Example 21 (mainly composed of 2-hydroxy-3,5-dinitro-butyrophenone with the structure of 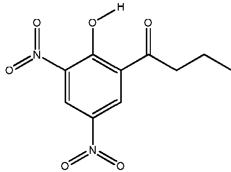 ) and 2 mmol of tributyl phosphine oxide were added to 20 mL of kerosene, and placed in a separatory funnel to form the extracting agent organic phase. Then, 20 mL of aqueous lithium-containing solution of B2 was added, shaken for 10 minutes, and then subjected to settlement and phase separation. The remaining aqueous phase R1 at the bottom was drained. The analysis results are shown in the table below.

| | c_{Li}/(g/L) | c_{Na}/(g/L) | c_{K}/(g/L) | pH |
|---|---|---|---|---|
| Lithium-containing solution B5 | 0.947 | 51.63 | - | 10.77 |
| Remaining aqueous phase R1 | 0.645 | 50.35 | - | 8.66 |

The extraction rates for lithium and sodium ions are 31.9% and 2.5%, respectively. The separation factor of Li/Na is 18.4.

### Example 23

Preparation of Extracting agent Organic Phase: 2 mmol of the synthesis product of Example 21 (mainly composed of 2-hydroxy-3,5-dinitro-butyrophenone with the structure of 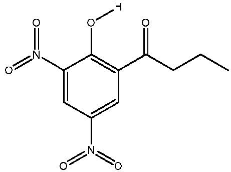 ) and 2 mmol of tributyl phosphine oxide were added to 20 mL of kerosene, and placed in a separatory funnel to form the extracting agent organic phase. Then, 20 mL of aqueous lithium-containing solution of B2 as well as 0.58 g of sodium carbonate were added, shaken for 10 minutes, and then subjected to settlement and phase separation. The remaining aqueous phase R1 at the bottom was drained. The analysis results are shown in the table below.

| | c_{Li}/(g/L) | c_{Na}/(g/L) | c_{K}/(g/L) | pH |
|---|---|---|---|---|
| Aqueous lithium-containing solution B2 + Na₂CO₃ | 0.947 | 51.90 | - | 10.89 |
| Remaining aqueous phase R1 | 0.189 | 46.96 | - | |

The extraction rates for lithium and sodium ions are 80.0% and 9.5%, respectively. The separation factor of Li/Na is 38.1.

### Example 24

The equilibrated aqueous solution from Example 12 was drained, leaving the organic mixture having lithium loaded. Then, 2 mL of water was added for washing. Afterwards, 0.3 mL of 0.6 M sulfuric acid aqueous solution was added, shaken for 3 minutes, and then subjected to settlement and phase separation. The remaining aqueous phase FC1 at the bottom was drained. The analysis results are shown in the table below.

| | c_{Li}/(g/L) | c_{Na}/(g/L) | c_{K}/(g/L) | pH |
|---|---|---|---|---|
| Lithium-containing solution FC1 obtained from stripping | 1.752 | 1.28 | - | < 1 |

### Example 25

Preparation of Extracting agent Organic Phase: 20 mmol of 4-(2-ethylhexyl alkoxy)-2-hydroxydibenzophenone was dissolved in 40 mL of glacial acetic acid, and 1.5 mL of 68 wt.% concentrated nitric acid was added. The mixture was reacted at 60°C for 10 hours, then dried under reduced pressure at 50°C to obtain a solid powder product (mainly composed of the compound with the structure of 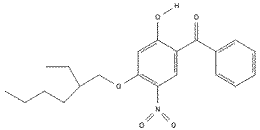 ). 2 mmol of the product and 2 mmol of trioctyl phosphine oxide were added together to 20 mL of kerosene, and placed in a separatory funnel to form the extracting agent organic phase. 20 mL of the lithium-containing solution of B2 was added, shaken to be fully dissolved to form a clear aqueous solution, and subsequently placed in a separatory funnel, shaken for 10 minutes, and then subjected to settlement and phase separation. The remaining aqueous phase R1 at the bottom were released. Again, 20 mL of the lithium-containing solution of B2 was added, shaken to be fully dissolved to form a clear aqueous solution, and subsequently placed in a separatory funnel, shaken for 10 minutes, and then subjected to settlement and phase separation. The bottom remaining aqueous phase R2 were released. Still again, 20 mL of the lithium-containing solution of B2 was added, shaken to be fully dissolved to form a clear aqueous solution, and subsequently placed in a separatory funnel, shaken for 10 minutes, and then subjected to settlement and phase separation. The bottom remaining aqueous phase R3 were released. The above process permits to obtain as much as possible lithium through the extracting agent organic phase. Afterward, 2 mL of water was added for washing, subjected to settlement and phase separation, and the washing liquid was drained. Then, 2 mL of 0.6 M sulfuric acid aqueous solution was added, shaken for 5 min for stripping, and the remaining aqueous phase FC1 at the bottom was drained. The analysis results are shown in the table below.

| | c_{Li}/(g/L) | c_{Na}/(g/L) | c_{K}/(g/L) | pH |
|---|---|---|---|---|
| Lithium-containing solution B2 | 0.947 | 51.90 | - | 10.77 |
| Remaining aqueous phase R1 | 0.616 | 49.71 | - | 8.44 |
| Remaining aqueous phase R2 | 0.770 | 50.21 | - | 9.26 |
| Remaining aqueous phase R3 | 0.856 | 51.51 | - | 9.61 |
| Remaining aqueous phase FC1 | 3.842 | 2.64 | - | - |

### Example 26

The extraction and stripping experiment described in Example 25 was repeated six times. After each extraction was completed, 0.08 mL of 10 M H₂SO₄ was added to the previous stripping solution FC1, and then the FC1 supplemented with acid was used for another stripping, thereby obtaining the stripping solution FC1 rich in lithium. The sample analysis results are as shown in the table below.

| | c_{Li}/(g/L) | c_{Na}/(g/L) | c_{K}/(g/L) | pH |
|---|---|---|---|---|
| Remaining aqueous phase FC1 | 19.21 | 14.55 | - | - |

### Example 27

Preparation of Extracting agent Organic Phase: 4 mmol of the synthesis product of Example 25 (mainly composed of the compound with the structure of 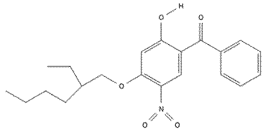 ) and 8 mmol of tributyl phosphine oxide were added to 40 mL of kerosene, and placed in a separatory funnel to form the extracting agent organic phase. Then, 50 mL of aqueous lithium-containing solution of B6 was added, shaken for 10 minutes, and then subjected to settlement and phase separation. The remaining aqueous phase R1 at the bottom was drained. Afterward, 4 mL of water was added for washing, subjected to settlement and phase separation, and the washing liquid was drained. Then, 1.5 mL of 0.6 M sulfuric acid aqueous solution was added, shaken for 5 min for stripping, and the remaining aqueous phase FC1 at the bottom was drained. The analysis results are shown in the table below.

| | c_{Li}/(g/L) | c_{Na}/(g/L) | c_{K}/(g/L) | pH |
|---|---|---|---|---|
| Lithium-containing solution B6 | 0.854 | 78.03 | - | 10.25 |
| Remaining aqueous phase R1 | 0.483 | 65.88 | - | - |
| Remaining aqueous phase FC1 | 3.324 | 1.92 | - | |

### Example 28

Preparation of Extracting agent Organic Phase: 1 mmol of 2,2'-dihydroxy-4,4'-dimethoxydibenzophenone (with the structure of 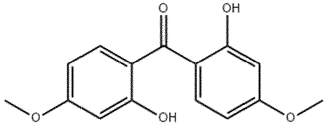 ) and 2 mmol of trioctyl phosphine oxide were added to 10 mL of cyclohexane, and placed in a separatory funnel, shaken until the solids were fully dissolved to form the extracting agent organic phase. Then, 4 mL of aqueous lithium-containing solution of B1 was added, shaken at 18°C for 10 minutes, and then subjected to settlement and phase separation. The remaining aqueous phase R1 at the bottom was drained. The analysis results are shown in the table below.

| | c_{Li}/(g/L) | c_{Na}/(g/L) | c_{K}/(g/L) | pH |
|---|---|---|---|---|
| Lithium-containing solution B1 | 0.807 | 56.12 | None | 11.48 |
| Remaining aqueous phase R1 | 0.132 | 54.23 | None | - |

The extraction rates for lithium and sodium ions are 83.6% and 3.4%, respectively. The separation factor of Li/Na is 146.7.

### Example 29

Preparation of Extracting agent Organic Phase: 1 mmol of 2-hydroxydibenzophenone (with the structure of 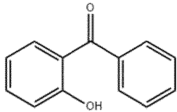 ) and 1 mmol of trioctyl phosphine oxide were added to 10 mL of cyclohexane, and placed in a separatory funnel, shaken until the solids were fully dissolved to form the extracting agent organic phase. Then, 4 mL of 0.5 mol/L NaOH was added, shaken for 30 minutes, and then subjected to settlement and phase separation. The bottom remaining aqueous phase was drained. Further, a aqueous lithium-containing solution of B2 was added, shaken at room temperature for 10 minutes, and then subjected to settlement and phase separation. The remaining aqueous phase R1 at the bottom was drained. The analysis results are shown in the table below.

| | c_{Li}/(g/L) | c_{Na}/(g/L) | c_{K}/(g/L) | pH |
|---|---|---|---|---|
| Lithium-containing solution B2 | 0.947 | 51.63 | None | 10.77 |
| Remaining aqueous phase R1 | 0.214 | 49.31 | None | - |

The extraction rates for lithium and sodium ions are 73.3% and 2.32%, respectively. The separation factor of Li/Na is 72.8.

### Example 30

Preparation of Extracting agent Organic Phase: 1 mmol of 2-hydroxy-5-methoxyacetophenone (with the structure of 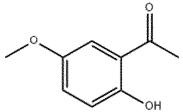 ) and 1 mmol of trioctyl phosphine oxide were added to 10 mL of heptane, and placed in a separatory funnel to form the extracting agent organic phase. Then, 4 mL of aqueous lithium-containing solution of B7 was added, shaken at 26°C for 10 minutes, and then subjected to settlement and phase separation. The remaining aqueous phase R1 at the bottom was drained. The analysis results are shown in the table below.

| | c_{Li}/(g/L) | c_{Na}/(g/L) | c_{K}/(g/L) | pH |
|---|---|---|---|---|
| Lithium-containing solution B7 | 0.833 | 55.60 | 5.37 | 12.05 |
| Remaining aqueous phase R1 | 0.331 | 49.56 | 4.99 | 10.30 |

The extraction rates for lithium, sodium and potassium ions are 60.26%, 10.86% and 7.08%, respectively. The separation factor of Li/Na is 12.4.

### Example 31

Preparation of Extracting agent Organic Phase: 1 mmol of 2-hydroxy-6-methoxyacetophenone (with the structure of 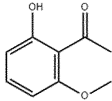 ) and 1 mmol of trioctyl phosphine oxide were added to 10 mL of heptane, and placed in a separatory funnel to form the extracting agent organic phase. Then, 4 mL of aqueous lithium-containing solution of B7 was added, shaken at 26°C for 10 minutes, and then subjected to settlement and phase separation. The remaining aqueous phase R1 at the bottom was drained. The analysis results are shown in the table below.

| | c_{Li}/(g/L) | c_{Na}/(g/L) | c_{K}/(g/L) | pH |
|---|---|---|---|---|
| Lithium-containing solution B7 | 0.833 | 55.60 | 5.37 | 12.05 |
| Remaining aqueous phase R1 | 0.388 | 50.11 | 5.20 | - |

The extraction rates for lithium, sodium and potassium ions are 53.42%, 9.86% and 3.2%, respectively. The separation factor of Li/Na is 10.5.

### Example 32

Preparation of Extracting agent Organic Phase: 1 mmol of 2-hydroxy-5-benzyloxyacetophenone (with the structure of 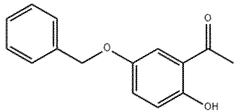 ) and 1 mmol of trioctyl phosphine oxide were added to 10 mL of heptane, and placed in a separatory funnel to form the extracting agent organic phase. Then, 5 mL of aqueous lithium-containing solution of B7 was added, shaken at 26°C for 10 minutes, and then subjected to settlement and phase separation. The remaining aqueous phase R1 at the bottom was drained. The analysis results are shown in the table below.

| | c_{Li}/(g/L) | c_{Na}/(g/L) | c_{K}/(g/L) | pH |
|---|---|---|---|---|
| Lithium-containing solution B7 | 0.833 | 55.60 | 5.37 | 12.05 |
| Remaining aqueous phase R1 | 0.445 | 51.26 | 5.01 | - |

The extraction rates for lithium, sodium and potassium ions are 46.6%, 7.8% and 6.7%, respectively. The separation factor of Li/Na is 10.3.

### Example 33

Preparation of Extracting agent Organic Phase: 1 mmol of 2-hydroxy-3,4-dimethoxyacetophenone (with the structure of 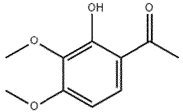 ) and 1 mmol of trioctyl phosphine oxide were added to 10 mL of heptane, and placed in a separatory funnel to form the extracting agent organic phase. Then, 5 mL of aqueous lithium-containing solution of B7 was added, shaken at 26°C for 10 minutes, and then subjected to settlement and phase separation. The remaining aqueous phase R1 at the bottom was drained. The analysis results are shown in the table below.

| | c_{Li}/(g/L) | c_{Na}/(g/L) | c_{K}/(g/L) | pH |
|---|---|---|---|---|
| Lithium-containing solution B7 | 0.833 | 55.60 | 5.37 | 12.05 |
| Remaining aqueous phase R1 | 0.416 | 48.96 | 4.95 | - |

The extraction rates for lithium, sodium and potassium ions are 50.06%, 11.94% and 7.8%, respectively. The separation factor of Li/Na is 7.4.

### Example 34

Preparation of Extracting agent Organic Phase: 1 mmol of 2-hydroxy-3-nitrocetophenone (with the structure of 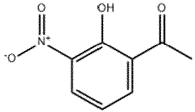 ) and 1 mmol of trioctyl phosphine oxide were added to 10 mL of heptane, and placed in a separatory funnel to form the extracting agent organic phase. Then, 5 mL of aqueous lithium-containing solution of B7 was added, shaken at 26°C for 10 minutes, and then subjected to settlement and phase separation. The remaining aqueous phase R1 at the bottom was drained. The analysis results are shown in the table below.

| | c_{Li}/(g/L) | c_{Na}/(g/L) | c_{K}/(g/L) | pH |
|---|---|---|---|---|
| Lithium-containing solution B7 | 0.833 | 55.60 | 5.37 | 12.05 |
| Remaining aqueous phase R1 | 0.056 | 51.06 | 5.12 | - |

The extraction rates for lithium, sodium and potassium ions are 93.28%, 8.17% and 4.5%, respectively. The separation factor of Li/Na is 156.

### Example 35

Preparation of Extracting agent Organic Phase: 1 mmol of 2-hydroxy-4-nitrocetophenone (with the structure of 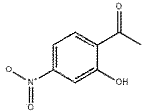 ) and 1 mmol of trioctyl phosphine oxide were added to 10 mL of heptane, and placed in a separatory funnel to form the extracting agent organic phase. Then, 5 mL of aqueous lithium-containing solution of B7 was added, shaken at 26°C for 10 minutes, and then subjected to settlement and phase separation. The remaining aqueous phase R1 at the bottom was drained. The analysis results are shown in the table below.

| | c_{Li}/(g/L) | c_{Na}/(g/L) | c_{K}/(g/L) | pH |
|---|---|---|---|---|
| Lithium-containing solution B7 | 0.833 | 55.60 | 5.37 | 12.05 |
| Remaining aqueous phase R1 | 0.069 | 51.66 | 5.21 | - |

The extraction rates for lithium, sodium and potassium ions are 91.72%, 7.09% and 3.0%, respectively. The separation factor of Li/Na is 145.

### Example 36

Preparation of Extracting agent Organic Phase: 1 mmol of 2-hydroxy-6-nitrocetophenone (with the structure of 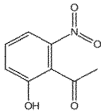 ) and 0.8 mmol of trioctyl phosphine oxide were added to 10 mL of heptane, and placed in a separatory funnel to form the extracting agent organic phase. Then, 5 mL of aqueous lithium-containing solution of B7 was added, shaken at 26°C for 10 minutes, and then subjected to settlement and phase separation. The remaining aqueous phase R1 at the bottom was drained. The analysis results are shown in the table below.

| | c_{Li}/(g/L) | c_{Na}/(g/L) | c_{K}/(g/L) | pH |
|---|---|---|---|---|
| Lithium-containing solution B7 | 0.833 | 55.60 | 5.37 | 12.05 |
| Remaining aqueous phase R1 | 0.157 | 51.03 | 4.87 | - |

The extraction rates for lithium, sodium and potassium ions are 81.1%, 8.2% and 9.3%, respectively. The separation factor of Li/Na is 48.

### Example 37

Preparation of Extracting agent Organic Phase: 1 mmol of 5-chloro-2-hydroxy-3-nitrocetophenone (with the structure of 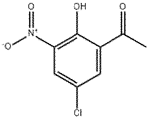 ) and 0.8 mmol of trioctyl phosphine oxide were added to 10 mL of D80 solvent oil, and placed in a separatory funnel to form the extracting agent organic phase. Then, 5 mL of aqueous lithium-containing solution of B7 was added, shaken at 26°C for 10 minutes, and then subjected to settlement and phase separation. The remaining aqueous phase R1 at the bottom was drained. The analysis results are shown in the table below.

| | c_{Li}/(g/L) | c_{Na}/(g/L) | c_{K}/(g/L) | pH |
|---|---|---|---|---|
| Lithium-containing solution B7 | 0.833 | 55.60 | 5.37 | 12.05 |
| Remaining aqueous phase R1 | 0.066 | 50.76 | 4.93 | - |

The extraction rates for lithium, sodium and potassium ions are 92.1%, 8.7% and 9.2%, respectively. The separation factor of Li/Na is 120.

### Example 38

Preparation of Extracting agent Organic Phase: 1 mmol of 2-hydroxy-5-chlorodibenzophenone (with the structure of 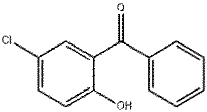 ) and 1 mmol of trioctyl phosphine oxide were added to 10 mL of D80 solvent oil, and placed in a separatory funnel to form the extracting agent organic phase. Then, 5 mL of aqueous lithium-containing solution of B7 was added, shaken at 26°C for 10 minutes, and then subjected to settlement and phase separation. The remaining aqueous phase R1 at the bottom was drained. The analysis results are shown in the table below.

| | c_{Li}/(g/L) | c_{Na}/(g/L) | c_{K}/(g/L) | pH |
|---|---|---|---|---|
| Lithium-containing solution B7 | 0.833 | 55.60 | 5.37 | 12.05 |
| Remaining aqueous phase R1 | 0.366 | 50.81 | 4.99 | - |

The extraction rates for lithium, sodium and potassium ions are 56.06%, 8.7% and 7.1%, respectively. The separation factor of Li/Na is 13.5.

### Example 39

Preparation of Extracting agent Organic Phase: 1 mmol of 2-hydroxy-5-iodoacetophenone (with the structure of 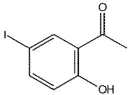 ) and 1 mmol of trioctyl phosphine oxide were added to 10 mL of D60 solvent oil, and placed in a separatory funnel to form the extracting agent organic phase. Then, 5 mL of aqueous lithium-containing solution of B7 was added, shaken at 26°C for 10 minutes, and then subjected to settlement and phase separation. The remaining aqueous phase R1 at the bottom was drained. The analysis results are shown in the table below.

| | c_{Li}/(g/L) | c_{Na}/(g/L) | c_{K}/(g/L) | pH |
|---|---|---|---|---|
| Lithium-containing solution B7 | 0.833 | 55.60 | 5.37 | 12.05 |
| Remaining aqueous phase R1 | 0.432 | 49.86 | 4.91 | - |

The extraction rates for lithium, sodium and potassium ions are 48%, 10.3% and 8.5%, respectively. The separation factor of Li/Na is 8.

### Example 40

Preparation of Extracting agent Organic Phase: 1 mmol of 2-hydroxy-5-fluoroacetophenone (with the structure of 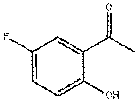 ) and 1 mmol of trioctyl phosphine oxide were added to 10 mL of D60 solvent oil, and placed in a separatory funnel to form the extracting agent organic phase. Then, 5 mL of aqueous lithium-containing solution of B7 was added, shaken at 26°C for 10 minutes, and then subjected to settlement and phase separation. The remaining aqueous phase R1 at the bottom was drained. The analysis results are shown in the table below.

| | c_{Li}/(g/L) | c_{Na}/(g/L) | c_{K}/(g/L) | pH |
|---|---|---|---|---|
| Lithium-containing solution B7 | 0.833 | 55.60 | 5.37 | 12.05 |
| Remaining aqueous phase R1 | 0.417 | 49.81 | 4.86 | - |

The extraction rates for lithium, sodium and potassium ions are 50%, 10.4% and 9.5%, respectively. The separation factor of Li/Na is 8.6.

### Example 41

Preparation of Extracting agent Organic Phase: 1 mmol of 2-hydroxy-2-phenylacetophenone (with the structure of 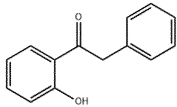 ) and 1 mmol of trioctyl phosphine oxide were added to 10 mL of cyclohexane, and placed in a separatory funnel, shaken until the solids were fully dissolved to form the extracting agent organic phase. Then, 4 mL of aqueous lithium-containing solution of B1 was added, shaken at 28°C for 10 minutes, and then subjected to settlement and phase separation. The remaining aqueous phase R1 at the bottom was drained, and 0.2 mL of it was diluted to 50 mL for component concentration detection using an Inductively Coupled Plasma Optical Emission Spectrometer (ICP) (the detection method is the same for subsequent examples). The results are shown in the table below.

| | c_{Li}/(g/L) | c_{Na}/(g/L) | c_{K}/(g/L) | pH |
|---|---|---|---|---|
| Lithium-containing solution B1 | 0.807 | 56.12 | None | 11.48 |
| Remaining aqueous phase R1 | 0.248 | 49.50 | None | 10.1 |

The extraction rates for lithium and sodium ions (*E* = (c_{B1} - c_{R1})/c_{B1} × 100%, throughout the application) are 69.3% and 11.8%, respectively. The separation factor of Li/Na is 16.8.

### Comparative Example 1 (β-diketone: benzoyl acetone)

Preparation of Extracting agent Organic Phase: 0.5 mmol of benzoyl acetone and 0.5 mmol of trioctyl phosphine oxide were added to 10 mL of cyclohexane, and placed in a separatory funnel, shaken until the solids were fully dissolved to form the extracting agent organic phase. Then, 4 mL of aqueous lithium-containing solution of B3 was added, shaken at room temperature for 10 minutes, and then subjected to settlement and phase separation. The remaining aqueous phase R1 at the bottom was drained. The analysis results are shown in the table below.

| | c_{Li}/(g/L) | c_{Na}/(g/L) | c_{K}/(g/L) | pH |
|---|---|---|---|---|
| Lithium-containing solution B3 | 0.937 | 119.10 | 43.03 | 9.60 |
| Remaining aqueous phase R1 | 0.440 | 112.63 | 41.79 | - |

The extraction rates for lithium and sodium ions are 53.0% and 5.43%, respectively. The separation factor of Li/Na is 19.6.

### Comparative Example 2 (β-diketone: benzoyl acetone)

Preparation of Extracting agent Organic Phase: 0.5 mmol of benzoyl acetone and 0.5 mmol of trioctyl phosphine oxide were added to 10 mL of cyclohexane, and placed in a separatory funnel, shaken until the solids were fully dissolved to form the extracting agent organic phase. Then, 4 mL of aqueous lithium-containing solution of B5 was added, shaken at room temperature for 10 minutes, and then subjected to settlement and phase separation. The remaining aqueous phase R1 at the bottom was drained. The analysis results are shown in the table below.

| | c_{Li}/(g/L) | c_{Na}/(g/L) | c_{K}/(g/L) | pH |
|---|---|---|---|---|
| Lithium-containing solution B5 | 0.261 | 39.98 | 7.61 | 9.49 |
| Remaining aqueous phase R1 | 0.200 | 38.20 | 7.52 | - |

The extraction rates for lithium and sodium ions are 23.4% and 4.45%, respectively. The separation factor of Li/Na is 6.54.

### Comparative Example 3 (β-diketone: 1,3-diphenyl-1,3-propanedione)

Preparation of Extracting agent Organic Phase: 0.5 mmol of 1,3-diphenyl-1,3-propanedione and 0.5 mmol of trioctyl phosphine oxide were added to 10 mL of cyclohexane, and placed in a separatory funnel, shaken until the solids were fully dissolved to form the extracting agent organic phase. Then, 4 mL of aqueous lithium-containing solution of B3 was added, shaken at room temperature for 10 minutes, and then subjected to settlement and phase separation. The remaining aqueous phase R1 at the bottom was drained. The analysis results are shown in the table below.

| | c_{Li}/(g/L) | c_{Na}/(g/L) | c_{K}/(g/L) | pH |
|---|---|---|---|---|
| Lithium-containing solution B3 | 0.937 | 119.10 | 43.03 | 9.60 |
| Remaining aqueous phase R1 | 0.377 | 115.32 | 43.21 | - |

The extraction rates for lithium and sodium ions are 59.76% and 3.17%, respectively. The separation factor of Li/Na is 45.3.

### Comparative Example 4 (β-diketone: 1,3-diphenyl-1,3-propanedione)

Preparation of Extracting agent Organic Phase: 0.5 mmol of 1,3-diphenyl-1,3-propanedione and 0.5 mmol of trioctyl phosphine oxide were added to 10 mL of cyclohexane, and placed in a separatory funnel, shaken until the solids were fully dissolved to form the extracting agent organic phase. Then, 4 mL of aqueous lithium-containing solution of B5 was added, shaken at room temperature for 10 minutes, and then subjected to settlement and phase separation. The remaining aqueous phase R1 at the bottom was drained. The analysis results are shown in the table below.

| | c_{Li}/(g/L) | c_{Na}/(g/L) | c_{K}/(g/L) | pH |
|---|---|---|---|---|
| Lithium-containing solution B3 | 0.261 | 39.98 | 7.61 | 9.49 |
| Remaining aqueous phase R1 | 0.186 | 38.12 | 7.60 | - |

The extraction rates for lithium and sodium ions are 28.73% and 4.65%, respectively. The separation factor of Li/Na is 8.3.

### Comparative Example 5 (β-diketone: stearoylbenzoylmethane)

Preparation of Extracting agent Organic Phase: 0.5 mmol of stearoylbenzoylmethane and 0.5 mmol of trioctyl phosphine oxide were added to 10 mL of cyclohexane, and placed in a separatory funnel, shaken until the solids were fully dissolved to form the extracting agent organic phase. Then, 4 mL of aqueous lithium-containing solution of B3 was added, shaken at room temperature for 10 minutes, and then subjected to settlement and phase separation. The remaining aqueous phase R1 at the bottom was drained. The analysis results are shown in the table below.

| | c_{Li}/(g/L) | c_{Na}/(g/L) | c_{K}/(g/L) | pH |
|---|---|---|---|---|
| Lithium-containing solution B3 | 0.937 | 119.10 | 43.03 | 9.60 |
| Remaining aqueous phase R1 | 0.577 | 113.57 | 41.03 | - |

The extraction rates for lithium and sodium ions are 38.42% and 4.64%, respectively. The separation factor of Li/Na is 12.8.

### Comparative Example 6 (β-diketone: 4,4,4-trifluoro-1-(4-tolyl)-1,3- butanedione)

Preparation of Extracting agent Organic Phase: 0.5 mmol of 4,4,4-trifluoro-1-(4-tolyl)-1,3- butanedione and 0.5 mmol of trioctyl phosphine oxide were added to 10 mL of cyclohexane, and placed in a separatory funnel, shaken until the solids were fully dissolved to form the extracting agent organic phase. Then, 4 mL of aqueous lithium-containing solution of B3 was added, shaken at room temperature for 10 minutes, and then subjected to settlement and phase separation. The remaining aqueous phase R1 at the bottom was drained. The analysis results are shown in the table below.

| | c_{Li}/(g/L) | c_{Na}/(g/L) | c_{K}/(g/L) | pH |
|---|---|---|---|---|
| Lithium-containing solution B3 | 0.937 | 119.10 | 43.03 | 9.60 |
| Remaining aqueous phase R1 | 0.177 | 115.17 | 42.11 | - |

The extraction rates for lithium and sodium ions are 81.11% and 3.30%, respectively. The separation factor of Li/Na is 125.8.

### Comparative Example 7 (1-phenyl-3-methyl-4-benzoyl-5-pyrazolone)

Preparation of Extracting agent Organic Phase: 1 mmol of 1-phenyl-3-methyl-4-benzoyl-5-pyrazolone and 1 mmol of trioctyl phosphine oxide were added to 10 mL of cyclohexane, and placed in a separatory funnel, shaken until the solids were fully dissolved to form the extracting agent organic phase. Then, 4 mL of aqueous lithium-containing solution of B3 was added, shaken at room temperature for 10 minutes, and then subjected to settlement and phase separation. The remaining aqueous phase R1 at the bottom was drained. The analysis results are shown in the table below.

| | c_{Li}/(g/L) | c_{Na}/(g/L) | c_{K}/(g/L) | pH |
|---|---|---|---|---|
| Lithium-containing solution B3 | 0.937 | 119.10 | 43.03 | 9.60 |
| Remaining aqueous phase R1 | 0.925 | 117.80 | 42.96 | - |

The extraction rates for lithium and sodium ions are 1.28% and 1.09%, respectively. The separation factor of Li/Na is 1.17.

### Comparative Example 8 (1-phenyl-3-methyl-4-benzoyl-5-pyrazolone)

Preparation of Extracting agent Organic Phase: 1 mmol of 1-phenyl-3-methyl-4-benzoyl-5-pyrazolone and 1 mmol of trioctyl phosphine oxide were added to 10 mL of cyclohexane, and placed in a separatory funnel, shaken until the solids were fully dissolved to form the extracting agent organic phase. Then, 4 mL of aqueous lithium-containing solution of B3 was added, shaken at room temperature for 10 minutes, and then subjected to settlement and phase separation. The remaining aqueous phase R1 at the bottom was drained. The analysis results are shown in the table below.

| | c_{Li}/(g/L) | c_{Na}/(g/L) | c_{K}/(g/L) | pH |
|---|---|---|---|---|
| Lithium-containing solution B1 | 0.807 | 56.12 | None | 11.48 |
| Remaining aqueous phase R1 | 0.747 | 53.65 | None | - |

The extraction rates for lithium and sodium ions are 7.43% and 4.40%, respectively. The separation factor of Li/Na is 1.74.

Comparing the Comparative Examples with the Examples, it can be seen that the structure proposed by the present invention can effectively replace β-diketone lithium extracting agents. When the phenyl ring of the compound of Formula (I) is unsubstituted, the effect is comparable to that of β-diketones without trifluoromethyl group, which normally requires a high alkalinity condition to achieve a high lithium extraction rate, but the former has lower manufacturing cost. When the moieties attached to the phenyl ring of the compound of Formula (I) comprise halogen or nitro group, the effect can be comparable to that of β-diketones with trifluoromethyl group, which can achieve a high lithium extraction rate even under lower alkalinity condition, but the former still have a much lower manufacturing cost than that of the latter. Therefore, the variation in the substituents R1 to R5 of the compound of Formula (I) of the present invention will significantly affect its lithium extraction capability, lithium/sodium and lithium/potassium separation capabilities the alkalinity required for lithium extraction, material cost, and process cost.

### Solution loss rate

The solution loss rate is evaluated through solubility in water. The solubility in pure water at room temperature (the solubility of the lithium extracting agent of the present invention is measured using UV-Vis absorption spectroscopy; and data of the Comparative Examples is sourced from public literature materials at www.chemicalbook.com):
4-octyloxy-3-nitro-2-hydroxydibenzophenone 0.01 g/L
4- octyloxy-2-hydroxydibenzophenone <0.01 g/L
4-pentyloxy-2-hydroxydibenzophenone 0.05 g/L
Salicylaldehyde (with the structure of 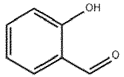 ), solubility of 4.9 g/L
2-hydroxyacetophenone (with the structure of 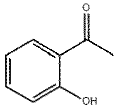 ), solubility of 0.2 g/L
2,4-dihydroxydibenzophenone (with the structure of 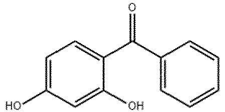 ), solubility of 0.7 g/L [J. Chem. Eng. Data 2008, 53, 8, 1996-1998]
2-hydroxy-4-methoxydibenzophenone (with the structure of 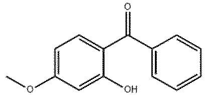 ), solubility of < 1.0 g/L
2-hydroxy-4-octyloxy-5-nitrodibenzophenone (with the structure of 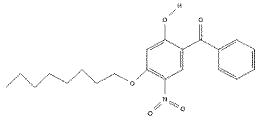 ), solubility of 0.01 g/L
Comparative Example: benzoyltrifluoroacetone (β-diketone) 0.24 g/L
Comparative Example: benzoyl acetone (β-diketone) 0.38 g/L

From the Example, it can be seen that the typical lithium extracting agent disclosed herein has much lower solution loss than that of β-diketones. In particular, 4-octyloxy-5-nitro-2-hydroxydibenzophenone which has a comparable lithium extraction effect to benzoyltrifluoracetone has an extremely low solubility in pure water, i.e., two orders of magnitude lower than that of benzoyltrifluoracetone.

The above examples are merely exemplary embodiments of the present invention and are not intended to limit the scope of protection of the present invention. The scope of protection of the present invention is determined by the appended claims.

## Claims

1. A compound of Formula (I) as lithium extracting agent, 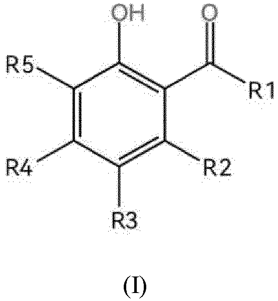
wherein R1 is H, C₁₋₈ alkyl, C₃₋₆ cycloalkyl, or phenyl, where the C₁₋₈ alkyl, C₃₋₆ cycloalkyl, or phenyl is optionally substituted with one or more substituents selected from the group consisting of hydroxyl, halogen, C₁₋₆ alkyl, C₁₋₆ alkoxy, or nitro, and the C₁₋₆ alkyl, C₁₋₆ alkoxy is optionally further substituted with one or more substituents selected from the group consisting of hydroxyl, halogen, C₁₋₆ alkyl, C₁₋₆ alkoxy, or nitro; and
R2, R3, R4 and R5 are each independently hydrogen, halogen, nitro, benzyloxy, C₁₋₆ alkyl, or C₁₋₁₆ alkoxy, where the benzyloxy, C₁₋₆ alkyl, or C₁₋₁₆ alkoxy is optionally substituted with one or more substituents selected from the group consisting of hydroxyl, halogen, C₁₋₆ alkyl, C₁₋₆ alkoxy, and nitro.

2. The compound of Formula (I) according to Claim 1, wherein R1 is C₁₋₆ alkyl or phenyl, where the C₁₋₆ alkyl or phenyl is optionally substituted with one or more substituents selected from the group consisting of hydroxyl, halogen, C₁₋₆ alkyl, phenyl, or C₁₋₆ alkoxy.

3. The compound of Formula (I) according to any one of Claims 1-2, wherein at least one of R2, R3, R4 and R5 is nitro.

4. The compound of Formula (I) according to any one of Claims 1-3, wherein at least one of R2, R3, R4 and R5 is benzyloxy, or unsubstituted or halogen substituted C₁₋₁₆ alkoxy.

5. The compound of Formula (I) according to Claim 4, wherein at least one of R2, R3, R4 and R5 is unsubstituted or halogen substituted C₆₋₁₆ alkoxy.

6. The compound of Formula (I) according to any one of Claims 1-5, wherein at least one of R2, R3, R4 and R5 is halogen.

7. The compound of Formula (I) according to any one of Claims 1-6, wherein R2 is H, nitro, or methoxy.

8. The compound of Formula (I) according to any one of Claims 1-7, wherein R3 is H, nitro, methoxy, halogen, or benzyloxy.

9. The compound of Formula (I) according to any one of Claims 1-8, wherein R4 is H, nitro, C₁₋₁₆ alkoxy, or benzyloxy.

10. The compound of Formula (I) according to any one of Claims 1-9, wherein R5 is H, nitro, or methoxy.

11. The compound of Formula (I) according to any one of Claims 1-10, wherein
R1 is methyl, ethyl, propyl, or phenyl; and/or
R2 is hydrogen, nitro, or methoxy; and/or
R3 is hydrogen, halogen, methoxy, or nitro; and/or
R4 is C₁₋₁₆ alkoxy; and/or
R5 is hydrogen, methoxy, and nitro.

12. The compound of Formula (I) according to Claim 1, selected from: 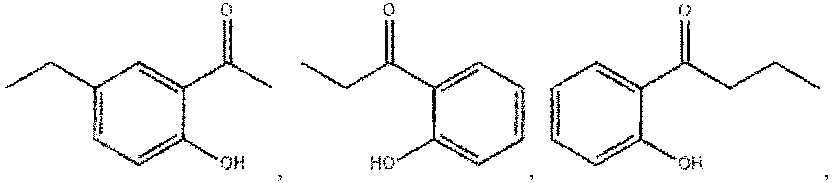 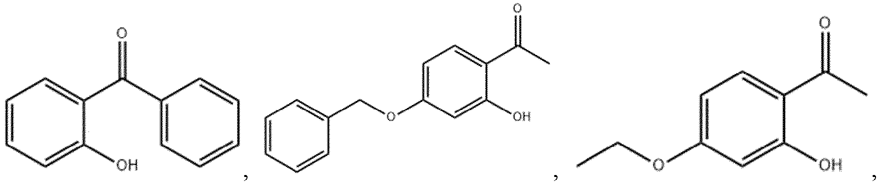 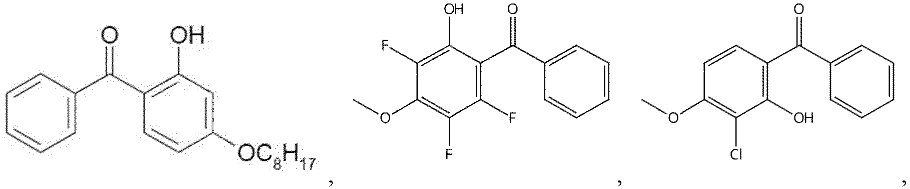 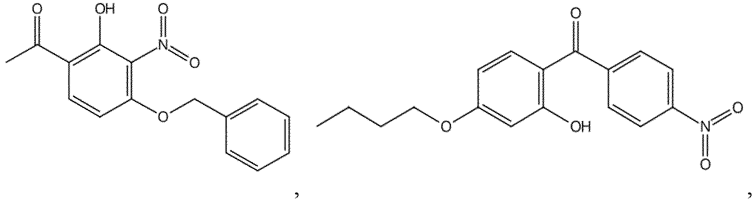 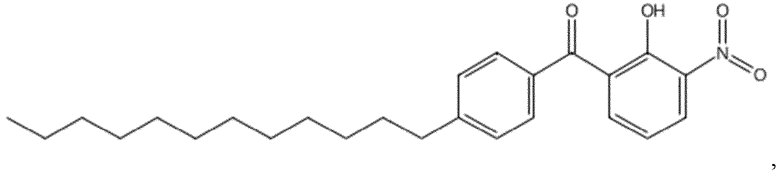 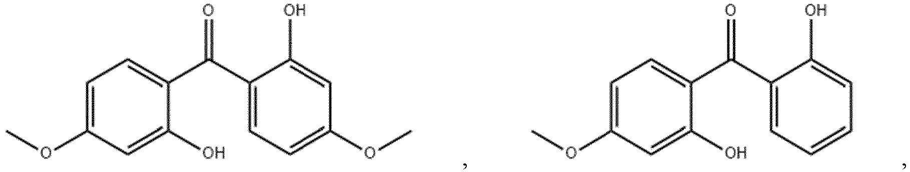 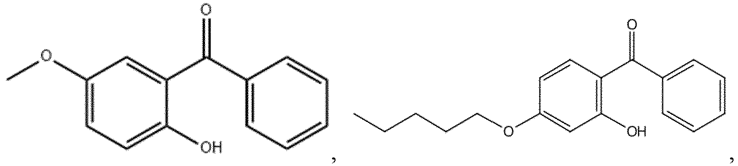 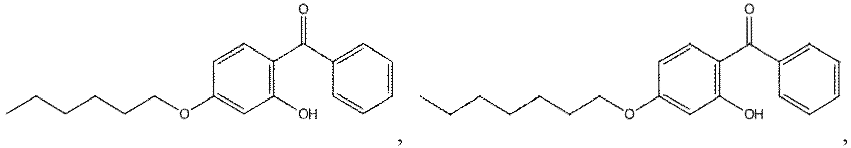 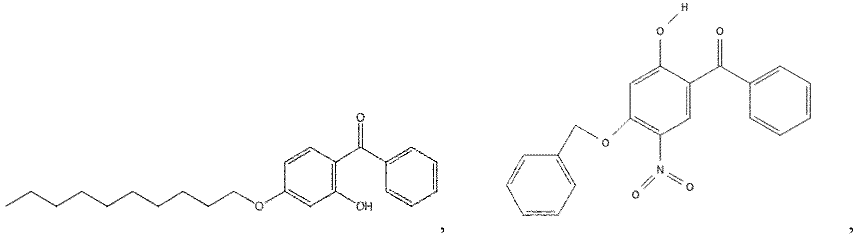 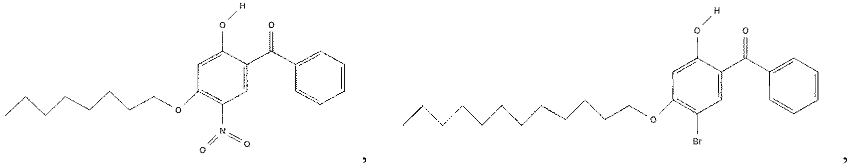 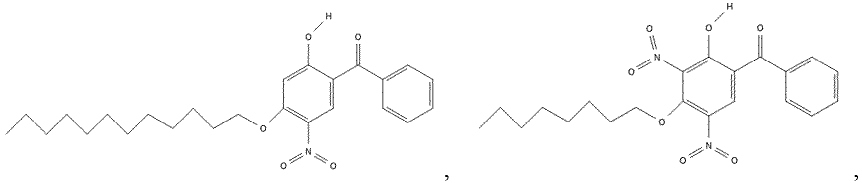 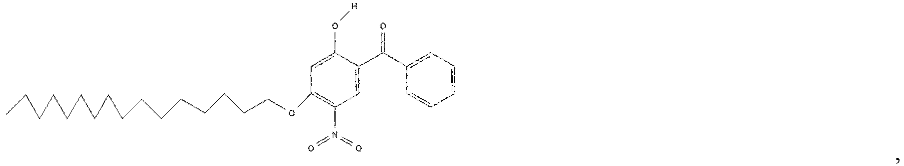 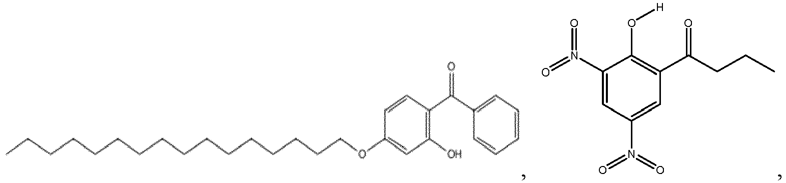 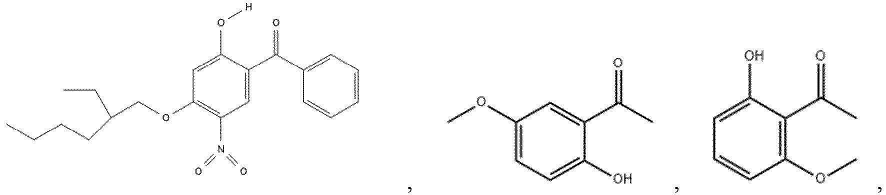 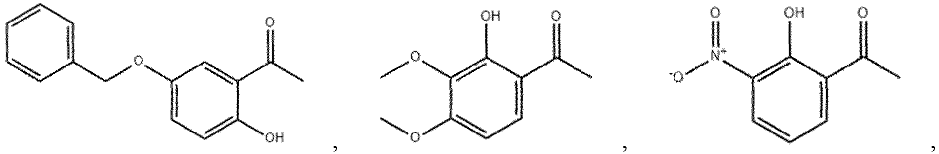 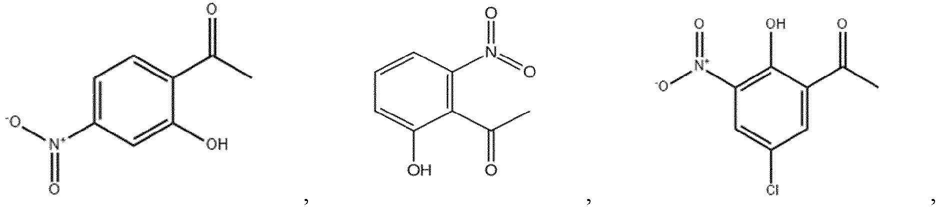 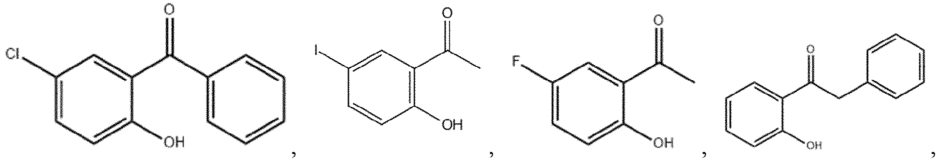 and

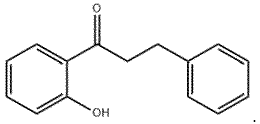

13. Use of the compound of Formula (I) according to any one of Claims 1-12 in lithium extraction as lithium extracting agent.

14. A liquid organic mixture for extracting lithium by means of extraction, comprising:
one or more of the compound of Formula (I) according to any of Claims 1-12,
a synergist, and
optionally a diluent.

15. The liquid organic mixture for extracting lithium by means of extraction according to Claim 14, wherein
the synergist is one or more of tributyl phosphate, trioctyl phosphate, tris(2-ethylhexyl) phosphate, trihexyl phosphate, tripentyl phosphate, butyl dibutyl phosphate, dibutyl butyl phosphate, methyl di-sec-octyl phosphate, methyl di-isooctyl phosphate, isopropyl di-isooctyl phosphate, methyl di-isopentyl phosphate, triphenyl phosphine oxide, diphenyl benzyl phosphine oxide, diphenyl(2-hydroxybenzyl) phosphine oxide, 2,5-dihydroxyphenyl(diphenyl) phosphine oxide, trioctyl phosphine oxide (or Cyanex921), trialkyl phosphine oxide TRPO (or Cyanex923).

16. The liquid organic mixture for extracting lithium by means of extraction according to Claim 14, or 15, wherein
the diluent is one or more of kerosene, D80 solvent oil, D60 solvent oil, heptane, cyclohexane, octane, dodecane, petroleum ether, xylene, anisole, methyl isobutyl ketone toluene, octanone 5-nonanone isopentanol, butanol, halobenzene.

17. The liquid organic mixture for extracting lithium by means of extraction according to any one of Claims 14-16, wherein the compound of Formula (I) has a total concentration of 0.03 mol/L to 1.0 mol/L in the liquid organic mixture for extracting lithium by means of extraction; the synergist has a concentration of 0.01 mol/L to 2.0 mol/L in the liquid organic mixture for extracting lithium by means of extraction; and the molar ratio of the compound of Formula (I) to the synergist is 3: 1 to 0.5:1.

18. A method for lithium extraction, comprising:
contacting the liquid organic mixture for extracting lithium by means of extraction according to any one of Claims 14-17 with an aqueous solution containing lithium ions, allowing the lithium ions from the aqueous solution to enter an organic layer by liquid-liquid phase separation, and collecting the organic layer.

19. The method according to Claim 18, wherein the aqueous solution containing lithium ions has a pH 7 to 13 before contact with the liquid organic mixture for extracting lithium by means of extraction.

20. The method according to Claim 19, wherein the alkalinity of the aqueous solution containing lithium ions is adjusted by adding a hydroxide, a carbonate, a phosphate, or a borate of alkali metal or ammonium .

21. The method according to Claim 19, wherein the aqueous solution containing lithium ions is an alkaline natural brine; where an alkalinity of the alkaline natural brine is optionally further adjusted by adding a hydroxide, a carbonate, a phosphate, or a borate of alkali metal or ammonium.

22. The method according to any one of Claims 18 to 20, wherein the aqueous solution containing lithium ion is a mother liquor from production of lithium carbonate and/or a mother liquor from production of lithium phosphate.

23. The method according to Claim 20, wherein the hydroxide of alkali metal or ammonium is one or more of sodium hydroxide, potassium hydroxide, and ammonium hydroxide; the carbonate is one or more of sodium carbonate and potassium carbonate; the phosphate is one or more of trisodium phosphate, tripotassium phosphate, triammonium phosphate, disodium hydrogen phosphate, dipotassium hydrogen phosphate, and diammonium hydrogen phosphate; and the borate is one or more of sodium metaborate, potassium metaborate, ammonium metaborate, sodium tetraborate, potassium tetraborate, and ammonium tetraborate.

24. The method according to Claim 18, wherein the aqueous solution containing lithium ions has a pH of 5 to 8 before contact with the liquid organic mixture for extracting lithium by means of extraction; and the liquid organic mixture for extracting lithium by means of extraction is previously saponified with one or more of sodium hydroxide, potassium hydroxide, sodium carbonate, potassium carbonate, sodium bicarbonate, or potassium bicarbonate aqueous solution before contact with the aqueous solution containing lithium ions.

25. The method according to any one of Claims 18 to 24, wherein a volume ratio of the aqueous solution containing lithium ions to the liquid organic mixture for extracting lithium by means of extraction is 30:1 to 1:30; and/or the aqueous solution containing lithium ions has a lithium concentration of 0.01 mol/L to 2.0 mol/L.

26. The method according to any one of Claims 18 to 25, further comprising a step of contacting a stripping agent with the organic layer to obtain a lithium-containing stripping agent.

27. The method according to Claim 26, wherein the stripping agent is an aqueous solution of acid.

28. The method according to Claim 27, wherein the stripping agent is an aqueous solution of one or more of carbonic acid, sulfuric acid, sulfurous acid, hydrochloric acid, nitric acid, and phosphoric acid; and/or, in the stripping agent, the concentration of hydrogen ions is 0.05 mol/L to 6 mol/L; and the volume ratio of the stripping agent to the organic layer is 1: 1 to 1:50.

29. The method according to Claim 26, wherein the lithium concentration in the stripping agent after contact with the organic layer is 0.05 mol/L to 10 mol/L.

30. The method according to any one of Claims 18 to 29, wherein an contact time of the liquid organic mixture for extracting lithium by means of extraction and the aqueous solution containing lithium ions is 3 to 30 minutes.

31. The method according to any one of Claims 26 to 30, wherein an contact time of the stripping agent and the organic layer is 3 to 30 minutes.

32. The method according to any one of Claims 18 to 31, wherein the contact is carried out in a mixer-settler, centrifugal extractor, or extraction column.

33. The method according to any one of Claims 18 to 32, wherein the liquid organic mixture for extracting lithium by means of extraction is contact with the same aqueous solution containing lithium ions one or more times.

34. The method according to any one of Claims 26 to 33, wherein the lithium-containing stripping agent is supplemented with a concentrated acid after contact with the organic layer, and then reused as stripping agent to contact the organic layer; and the concentrated acid is 6~12 mol/L of hydrochloric acid, 6~18 mol/L of sulfuric acid, 6~14 mol/L of nitric acid, or 6~15 mol/L of phosphoric acid.

35. The method according to any one of Claims 26 to 34, further comprising an operation of concentrating lithium by evaporating water from the lithium-containing stripping agent, and/or further precipitating lithium carbonate from the lithium-containing stripping agent.
